(19) Europäisches Patentamt
European Patent Office
Office européen des brevets

(11) EP 1 318 143 A1

(12) **EUROPEAN PATENT APPLICATION**
published in accordance with Art. 158(3) EPC

(43) Date of publication:
**11.06.2003 Bulletin 2003/24**

(21) Application number: **01965600.8**

(22) Date of filing: **13.09.2001**

(51) Int Cl.[7]: **C07C 311/48**, C07C 317/14, C07D 291/02, C07F 5/00, C07F 9/53, C09K 11/06, G02B 1/04, G02B 6/00

(86) International application number:
**PCT/JP01/07931**

(87) International publication number:
**WO 02/022566 (21.03.2002 Gazette 2002/12)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **13.09.2000 JP 2000278207**

(71) Applicants:
- **Yanagida, Shozo**
**Hyogo 666-0133 (JP)**
- **Nissei Chemicals Inc.**
**Osaka-shi, Osaka 532-0001 (JP)**

(72) Inventors:
- **YANAGIDA, Shozo**
**Kawanishi-shi, Hyogo 666-0133 (JP)**
- **SOGABE, Kensaku**
**Suita-shi, Osaka 565-0851 (JP)**
- **HASEGAWA, Yasuchika**
**Toyonaka-shi, Osaka 560-0055 (JP)**

(74) Representative: **Jones, Helen Marjorie Meredith**
**Gill Jennings & Every,**
**Broadgate House,**
**7 Eldon Street**
**London EC2M 7LH (GB)**

(54) **RARE-EARTH TERNARY COMPLEX**

(57) A rare earth ternary complex represented by the following formula (1):

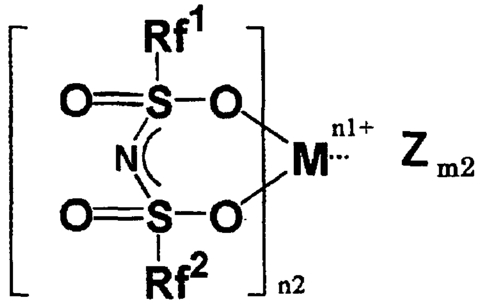

(1)

wherein M is a rare earth atom; n1 is 2 or 3; n2 is 2, 3, or 4; $Rf^1$ and $Rf^2$ are the same or different and are each a aliphatic group having 1 to 22 carbon atoms and containing no hydrogen atoms, an aromatic group containing no hydrogen atoms, or an aromatic heterocyclic group containing no hydrogen atoms; and Z is a ligand containing X, phosphorus, or nitrogen.

EP 1 318 143 A1

## Description

### TECHNICAL FIELD

This invention relates to a rare earth ternary complex, a composition comprising the rare earth ternary complex, and an optically functional material.

The rare earth ternary complex of the present invention is suitable, for example, as an optically functional material such as a luminescent material, a mechanoluminescence material, or the like, and can be used in optical fibers, lenses, or the like.

"Rare earth ternary complex" here refers to a rare earth complex formed by coordinating two organic compound molecules to a rare earth metal, or to a rare earth complex composed of three components, namely, a rare earth metal and two organic compounds.

### BACKGROUND ART

Various kinds of rare earth complexes have been developed in the past as optically functional materials. For instance, U.S. Patent 4,037,172 discloses a β-diketone rare earth complex as an optical conversion material. This publication discloses a rare earth complex in which hexafluoroacetylacetone (hereinafter referred to as HFA) is coordinated to europium. This complex has low luminescence intensity, so a large quantity of complex is required to obtain the necessary luminescence intensity.

An attempt has also been made at improving the optical functional characteristics of a rare earth complex by coordinating various types of photosensitizer to a rare earth complex. For instance, Japanese Unexamined Patent Publication 1989-256584 discloses a rare earth ternary complex having β-diketone and phosphine oxide as ligands. Said publication disclosed a ternary complex in which thenoyltrifluoroacetone and trioctylphosphine oxide are coordinated to terbium, for example.

However, a satisfactory level of luminescence characteristics cannot be achieved even with a rare earth complex having a photosensitizer such as phosphine oxide etc. as a ligand. Also, dispersibility in a polymer matrix does not reach a satisfactory level.

### DISCLOSURE OF THE INVENTION

It is an object of the present invention to provide a rare earth ternary complex that exhibits excellent luminescence characteristics.

The present inventors carried out extensive research to achieve the above object, and found that a rare earth ternary complex in which a specific organic hetero compound and a specific neutral ligand are coordinated exhibits remarkable higher luminescence intensity, beyond all expectations, as compared to known rare earth complexes.

Specifically, the present invention provides the following rare earth ternary complex, composition, and optically functional material.

1. A rare earth ternary complex represented by the following formula (1):

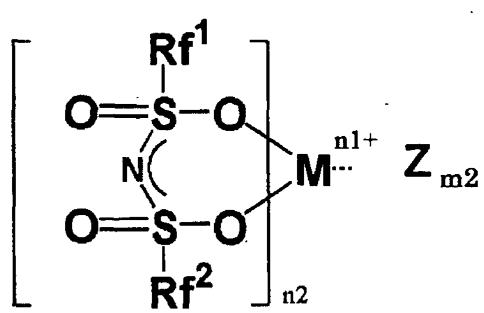

(1)

wherein M is a rare earth atom; n1 is 2 or 3; n2 is 2, 3, or 4; $Rf^1$ and $Rf^2$ are the same or different and are each an aliphatic group having 1 to 22 carbon atoms and containing no hydrogen atoms, an aromatic group containing no hydrogen atoms, or an aromatic heterocyclic group containing no hydrogen atoms; Z is at least one ligand selected from the group consisting of (A) to (D) below; m2 is an integer from 1 to 10 when Z is at least one

ligand selected from the group consisting of (A) to (C) below, and is an integer from 1 to 5 when Z is at least one ligand selected from among (D):

(A) a ligand represented by the following formula (A):

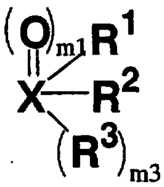
(A)

wherein $R^1$, $R^2$, and $R^3$ are the same or different and are each a hydrogen atom, a deuterium atom, an alkyl group having 1 to 20 carbon atoms, an alkyloxy group having 1 to 20 carbon atoms, an aromatic group, or an aryloxy group, and these groups may be substituted with deuterium, provided that at least one of $R^1$, $R^2$, and $R^3$ is an aromatic group or an aryloxy group; X is a phosphorus or sulfur atom; m1 is 0 or 1; and when X is a phosphorus atom, m3 is 1, and when X is a sulfur atom, m3 is 0,
(B) a ligand represented by the following formula (B):

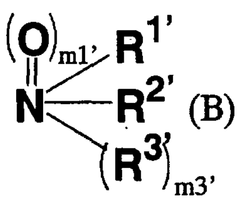
(B)

wherein $R^{1'}$, $R^{2'}$, and $R^{3'}$ are the same or different and are each a hydrogen atom, a deuterium atom, an alkyl group having 1 to 20 carbon atoms, an alkyloxy group having 1 to 20 carbon atoms, or an aromatic group, and these groups may be substituted with deuterium, provided that at least one of $R^{1'}$, $R^{2'}$, and $R^{3'}$ is an aromatic group; m1' is 0 or 1; and m3' is 1,
(C) a ligand which is a nitrogen-containing aromatic compound with monodentate coordination to M, and
(D) a ligand which is a nitrogen-containing aromatic compound with bidentate coordination to M.

2. The rare earth ternary complex according to the above-described item 1, wherein Z is a ligand represented by formula (A).

3. The rare earth ternary complex according to the above-described item 2, wherein formula (A) is as follows:

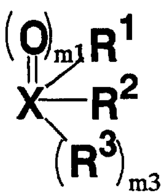
(A)

wherein $R^1$, $R^2$, and $R^3$ are the same or different and are each a hydrogen atom, an alkyl group having 1 to 20 carbon atoms, an alkyloxy group having 1 to 20 carbon atoms, an aromatic group, or an aryloxy group, and these groups may be substituted with deuterium, provided that at least one of $R^1$, $R^2$, and $R^3$ is an aromatic group or an aryloxy group; X is a phosphorus or sulfur atom; m1 is 0 or 1; and when X is a phosphorus atom, m3 is 1, and when X is a sulfur atom, m3 is 0.

4. The rare earth ternary complex according to the above-described item 1, which is obtainable by a preparing method comprising the step of mixing a rare earth complex represented by formula (2):

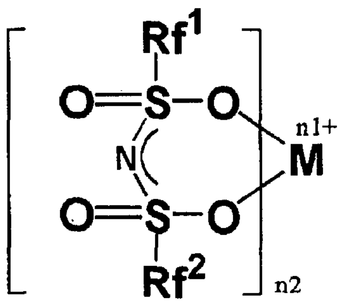

(2)

wherein M, n1, n2, $Rf^1$ and $Rf^2$ are as defined in the above-described item 1; and at least one compound selected from the group consisting of (A) to (D) below:

(A) a compound represented by the following formula (3) :

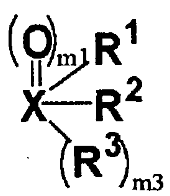

(3)

wherein $R^1$, $R^2$, and $R^3$ are the same or different and are each a hydrogen atom, a deuterium atom, an alkyl group having 1 to 20 carbon atoms, an alkyloxy group having 1 to 20 carbon atoms, an aromatic group, or an aryloxy group, and these groups may be substituted with deuterium, provided that at least one of $R^1$, $R^2$, and $R^3$ is an aromatic group or an aryloxy group; X is a phosphorus or sulfur atom; m1 is 0 or 1; and when X is a phosphorus atom, m3 is 1, and when X is a sulfur atom, m3 is 0,

(B) a compound represented by the following formula (B'):

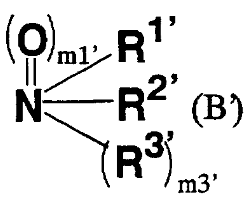

(B')

wherein $R^{1'}$, $R^{2'}$, and $R^{3'}$ are the same or different and are each a hydrogen atom, a deuterium atom, an alkyl group having 1 to 20 carbon atoms, an alkyloxy group having 1 to 20 carbon atoms, or an aromatic group, and these groups may be substituted with deuterium, provided that at least one of $R^{1'}$, $R^{2'}$, and $R^{3'}$ is an aromatic group; m1' is 0 or 1; and m3' is 1,

(C) a nitrogen-containing aromatic compound with monodentate coordination to M, and

(D) a nitrogen-containing aromatic compound with bidentate coordination to M

5. The rare earth ternary complex according to the above-described item 2, wherein at least one of $R^1$, $R^2$, and $R^3$ in formula (A) is an aromatic group.

6. The rare earth ternary complex according to the above-described item 2, wherein $R^1$, $R^2$, and $R^3$ in formula (A)

are phenyl groups.

7. The rare earth ternary complex according to the above-described item 1, wherein said complex is represented by formula (4):

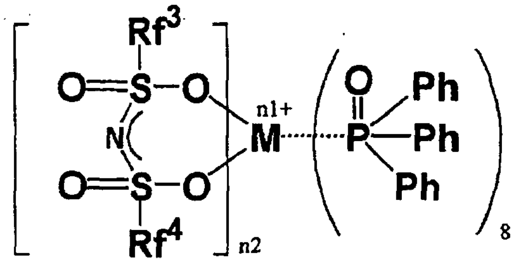

(4)

wherein M, n1, and n2 are as defined in the above-described item 1; $Rf^3$ and $Rf^4$ are the same or different and are each a perfluoroalkyl group having 1 to 4 carbon atoms; and Ph is a phenyl group.

8. The rare earth ternary complex according to the above-described item 1, wherein said complex is represented by formula (5):

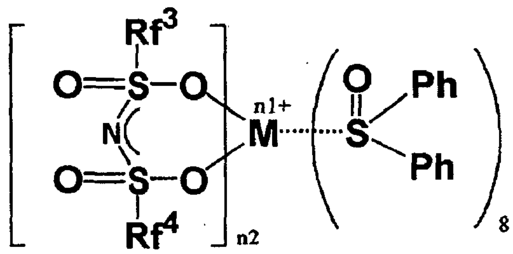

(5)

wherein M, n1, and n2 are as defined in the above-described item 1; $Rf^3$ and $Rf^4$ are the same or different and are each a perfluoroalkyl group having 1 to 4 carbon atoms; and Ph is a phenyl group.

9. The rare earth ternary complex according to the above-described item 1, 7, or 8, wherein M is a rare earth atom selected from the group consisting of Nd, Eu, Tb, and Yb.

10. A composition comprising:

a rare earth binary complex represented by formula (2):

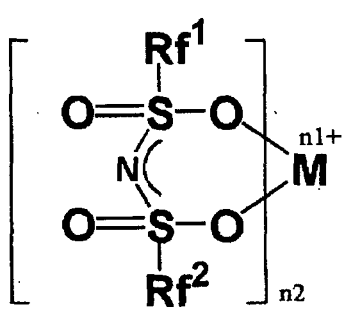

(2)

wherein M, n1, n2, $Rf^1$ and $Rf^2$ are as defined in the above-described item 1; and at least one compound selected from the group consisting of (A) to (D) below:

(A) a compound represented by the following formula (3):

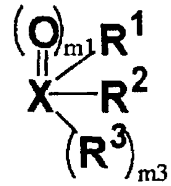

(3)

wherein $R^1$, $R^2$, and $R^3$ are the same or different and are each a hydrogen atom, a deuterium atom, an alkyl group having 1 to 20 carbon atoms, an alkyloxy group having 1 to 20 carbon atoms, an aromatic group, or an aryloxy group, and these groups may be substituted with deuterium, provided that at least one of $R^1$, $R^2$, and $R^3$ is an aromatic group or an aryloxy group; X is a phosphorus or sulfur atom; m1 is 0 or 1; and when X is a phosphorus atom, m3 is 1, and when X is a sulfur atom, m3 is 0,

(B) a compound represented by the following formula (B'):

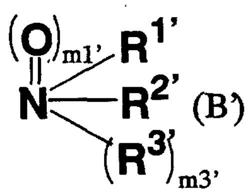

(B')

wherein $R^{1'}$, $R^{2'}$, and $R^{3'}$ are the same or different and are each a hydrogen atom, a deuterium atom, an alkyl group having 1 to 20 carbon atoms, an alkyloxy group having 1 to 20 carbon atoms, or an aromatic group, and these groups may be substituted with deuterium, provided that at least one of $R^{1'}$, $R^{2'}$, and $R^{3'}$ is an aromatic group; m1' is 0 or 1; and m3' is 1,

(C) a nitrogen-containing aromatic compound with monodentate coordination to M, and

(D) a nitrogen-containing aromatic compound with bidentate coordination to M.

11. The composition according to the above-described item 10, further comprising a solvent.

12. The composition according to the above-described item 10, further comprising a polymer matrix or a monomer which serves as the raw material for a polymer matrix.

13. An optically functional material comprising the rare earth ternary complex according to the above-described item 1.

14. An optically functional material comprising the composition according to the above-described item 10.

In the present invention, $Rf^1$ and $Rf^2$ are the same or different and are each an aliphatic group having 1 to 22 carbon atoms and containing no hydrogen atoms, an aromatic group containing no hydrogen atoms, or an aromatic heterocyclic group containing no hydrogen atoms. $Rf^3$ and $Rf^4$ are the same or different and are each a perfluoroalkyl group having 1 to 4 carbon atoms.

Examples of the aliphatic group having 1 to 22 carbon atoms and containing no hydrogen atoms include the following groups.

**[0012]** * Straight-chain or branched-chain perhalogenated alkyl groups such as perfluoroalkyl groups ($C_nF_{2n+1}$; n = 1 to 22), perchloroalkyl groups ($C_nCl_{2n+1}$; n = 1 to 22), and the like

Examples of the straight-chain or branched-chain perhalogenated alkyl groups include trichloromethyl, trifluoromethyl, pentachloroethyl, pentafluoroethyl, heptachloropropyl, heptafluoropropyl, heptachloroisopropyl, heptafluoroisopropyl, nonachlorobutyl, nonafluorobutyl, nonachloroisobutyl, nonafluoroisobutyl, undecachloropentyl, undecafluoropentyl, undecachloroisopentyl, undecafluoroisopentyl, tridecachlorohexyl, tridecafluorohexyl, tridecachloroisohexyl, tridecafluoroisohexyl, pentadecachloroheptyl, pentadecafluoroheptyl, pentadecachloroisoheptyl, pentadecafluoroisoheptyl, heptadecachlorooctyl, heptadecafluorooctyl, heptadecachloroisooctyl, heptadecafluoroisooctyl,

nonadecachlorononyl, nonadecafluorononyl, nonadecachloroisononyl, nonadecafluoroisononyl, heneicosachlorodecyl, heneicosafluorodecyl, heneicosachloroisodecyl, heneicosafluoroisodecyl, tricosachloroundecyl, tricosafluoroundecyl, tricosachloroisoundecyl, tricosafluoroisoundecyl, pentacosachlorododecyl, pentacosafluorododecyl, pentacosachloroisododecyl, pentacosafluoroisododecyl, heptacosachlorotridecyl, heptacosafluorotridecyl, heptacosachloroisotridecyl, heptacosafluoroisotridecyl, and the like. Other examples include nonafluoro-tert-butyl, nonachloro-tert-butyl and the like.

The straight-chain or branched-chain perhalogenated alkyl groups are preferably perchloroalkyl groups and perchlorofluoroalkyl groups, more preferably perfluoroalkyl groups.

The carbon number of the perhalogenated alkyl groups is usually 1 to 22, preferably 1 to 13, more preferably 1 to 10, particularly preferably 1 to 6, and the most preferably 1 to 4.

**[0016]** * Straight-chain or branched-chain perhalogenated alkenyl groups such as perfluoroalkenyl groups ($C_nF_{2n-1}$; n = 1 to 22), perchloroalkenyl groups ($C_nCl_{2n-1}$; n = 1 to 22), and the like;

Examples of the perhalogenated alkenyl groups include trifluorovinyl, trichlorovinyl, pentafluoroallyl, pentachloroallyl, pentafluoropropenyl, pentachloropropenyl, heptafluorobutenyl, heptachlorobutenyl, and the like. The perhalogenated alkenyl groups are preferably pentafluoroallyl, pentachloroallyl, and the like.

The carbon number of the perhalogenated alkenyl groups is usually 2 to 22, preferably 2 to 8, and more preferably 2 to 4.

**[0019]** * Straight-chain or branched-chain perhalogenated alkynyl groups having 2 to 22 carbon atoms such as perfluoroalkynyl groups, perchloroalkynyl groups, and the like;

Examples of the perhalogenated alkynyl groups include fluoroethynyl, chloroethynyl, 1-trifluoropropynyl, 1-trichloropropynyl, 2-trifluoropropynyl, 2-trichloropropynyl, and the like.

The carbon number of the perhalogenated alkenyl groups is usually 2 to 22, preferably 2 to 8, and more preferably 2 to 4.

**[0022]** * Perhalogenated cycloalkyl groups having 3 to 22 carbon atoms such as perfluorocycloalkyl ($C_nF_{2n-1}$; n = 3 to 22), perchlorocycloalkyl ($C_nCl_{2n-1;}$ n = 3 to 22), and the like;

Examples of the perhalogenated cycloalkyl groups include pentachlorocyclopropyl, pentafluorocyclopropyl, heptachlorocyclobutyl, heptafluorocyclobutyl, nonachlorocyclopentyl, nonafluorocyclopentyl, undecachlorocyclohexyl, undecafluorocyclohexyl, tridecachlorocycloheptyl, tridecafluorocycloheptyl, pentadecachlorocyclooctyl, pentadecafluorocyclooctyl, and the like.

The carbon number of the perhalogenated cycloalkyl groups is usually 3 to 22, preferably 3 to 8, and more preferably 3 to 6.

**[0025]** * Perhalogenated cycloalkenyl groups having 3 to 22 carbon atoms such as perfluorocycloalkenyl groups (for example, perfluorocyclopentenyl, perfluorocyclohexenyl etc.) perchlorocycloalkenyl groups, and the like

The carbon number of the perhalogenated cycloalkenyl groups is usually 3 to 22, preferably 3 to 8, and more preferably 3 to 6.

**[0027]** * Perhalogenated aralkyl groups such as perfluorobenzyl group, perchlorobenzyl group, perfluorophenethyl group, perchlorophenethyl group, and the like;

The perhalogenated aralkyl groups are preferably perfluoroaralkyl groups such as perfluorobenzyl group, perfluorophenethyl group, and the like.

Examples of the aromatic groups in "aromatic groups containing no hydrogen atoms" include phenyl, naphthyl, anthranyl, phenanthryl, pyrenyl and the like.

Examples of the aromatic heterocyclic groups in "aromatic heterocyclic groups containing no hydrogen atoms" include pyridyl, thienyl, pyrrolyl, pyrimidinyl, quinolyl, isoquinolyl, benzimidazolyl, benzopyranyl, indolyl, benzofuranyl, imidazolyl, pyrazolyl, biphenyl, and the like.

All of the hydrogen atoms in these aromatic groups and aromatic heterocyclic groups are substituted with substituents containing no hydrogen atoms, such as halogen atoms (fluorine atoms, chlorine atoms, bromine atoms, etc.), nitro groups, perhalogenated alkyl groups having 1 to 4 carbon atoms (trifluoromethyl, etc.), perhalogenated alkoxy groups having 1 to 4 carbon atoms (trifluoromethoxy, etc.), perhalogenated alkylcarbonyl groups having 2 to 5 carbon atoms (trifluoroacetyl, etc.), perhalogenated alkylenedioxy groups having 1 to 4 carbon atoms (difluoromethylenedioxy, etc.), perhalogenated alkenyl groups having 2 to 5 carbon atoms (perhalogenated vinyl, etc.), perhalogenated phenoxy groups, and perhalogenated alkylcarbonyloxy groups having 2 to 22 carbon atoms, and the like. Similarly, examples of the substituents for the aromatic groups and the aromatic heterocyclic groups containing no hydrogen atoms include cyano, nitroso, perhalogenated alkoxycarbonyl groups having 2 to 5 carbon atoms, and the like. These substituents may all be the same, or two or more kinds may be included. The above substituents are preferably halogen atoms, the perhalogenated alkyl groups, cyano groups, and nitroso groups, more preferably halogen atoms and perhalogenated alkyl groups.

Specific examples of the aromatic groups containing no hydrogen atoms include perfluorophenyl, perchlorophenyl, perfluoronaphthyl, perchloronaphthyl, perfluoroanthranyl, perchloroanthranyl, perfluorophenanthryl, perchlorophenanthryl, perchloropyrenyl, perfluoropyrenyl, perbromopyrenyl, and other such perhalogenated aromatic groups. Preferable examples include perfluorophenyl, perchlorophenyl, perfluoronaphthyl, perchloronaphthyl, perfluoroanthranyl, perchloroanthranyl, perfluorophenanthryl, perchlorophenanthryl, and the like.

Examples of the aromatic heterocyclic groups containing no hydrogen atoms include perhalogenated 2-pyridyl and other such perhalogenated aromatic heterocyclic groups.

One or more of the halogen atoms bonded to the aromatic ring of the perhalogenated aromatic group or to the aromatic hetero ring of the perhalogenated aromatic heterocyclic group may be substituted with substituents containing no hydrogen atoms, such as a cyano, nitro, nitroso, perhalogenated alkoxy having 1 to 4 carbon atoms, perhalogenated alkoxycarbonyl having 2 to 5 carbon atoms, perhalogenated alkylcarbonyloxy having 2 to 22 carbon atoms, or the like.

One or more of the halogen atoms bonded to the aromatic ring of the perhalogenated aralkyl group or the aromatic hetero ring may be substituted with substituents containing no hydrogen atoms, such as a cyano, nitro, nitroso, perhalogenated alkoxy having 1 to 4 carbon atoms, perhalogenated alkoxycarbonyl having 2 to 5 carbon atoms, perhalogenated alkylcarbonyloxy having 2 to 22 carbon atoms, or the like.

In $C_1$ to $C_{22}$ perhalogenated alkyl groups, $C_2$ to $C_{22}$ perhalogenated alkenyl groups, and $C_2$ to $C_{22}$ perhalogenated alkynyl groups, an ether, ester, or ketone structure may be produced by interposing one or more -O-, -COO, or -CO-groups between C-C single bonds at any position.

The perhalogenated alkyl groups or the perhalogenated aromatic groups are preferable as $Rf^1$ and $Rf^2$, and the perfluoroalkyl groups or the perfluoroaromatic groups are more preferable. When the chemical stability of the rare earth ternary complex is taken into account, it is particularly preferable for $Rf^1$ and $Rf^2$ to be the perhalogenated alkyl groups having 1 to 4 carbon atoms, and of these, the perfluoroalkyl groups having 1 to 4 carbon atoms are the most preferable. In view of the luminescence intensity, $Rf^1$ and $Rf^2$ are preferably the perhalogenated alkyl groups having 1 to 4 carbon atoms or perhalogenated aromatic groups, more preferably perfluoro-substituted aromatic groups such as a perfluorophenyl group.

Examples of the rare earth elements represented by M include La, Ce, Pr, Nd, Pm, Sm, Eu, Gd, Tb, Dy, Ho, Er, Tm, Yb, Lu, and other lanthanoids. Nd, Eu, Tb and Yb are preferable as M, Nd and Eu are more preferable, and Eu is particularly preferable.

n1 is 2 or 3. n2 is 2, 3, or 4.

When the rare earth ternary complex of the present invention is an anion, the cation that serves as the counter ion is not limited. Examples of cations include tetrabutylammonium ions, benzyltrimethylammonium ions, and other such quaternary ammonium ions; tetrabutylphosphonium ions and other such phosphonium ions and the like.

The rare earth ternary complex of the present invention has a ligand represented by Z in formula (1). The ligand Z is at least one ligand selected from the group consisting of the following (A) to (D).

(A) a ligand represented by the following formula (A):

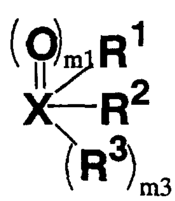
(A)

wherein $R^1$, $R^2$, and $R^3$ are the same or different and are each a hydrogen atom, a deuterium atom, an alkyl group having 1 to 20 carbon atoms, an alkyloxy group having 1 to 22 carbon atoms, an aromatic group, or an aryloxy group, and these groups may be substituted with deuterium; at least one of $R^1$, $R^2$, and $R^3$ is an aromatic group or an aryloxy group; X is a phosphorus or sulfur atom; m1 is 0 or 1; and when X is a phosphorus atom, m3 is 1, and when X is a sulfur atom, m3 is 0,

(B) a ligand represented by the following formula (B):

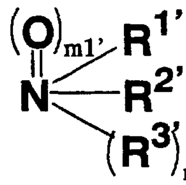 (B)

wherein R1', $R^{2'}$, and $R^{3'}$ are the same or different and are each a hydrogen atom, a deuterium atom, an alkyl group having 1 to 20 carbon atoms, an alkyloxy group having 1 to 20 carbon atoms, or an aromatic group, and these groups may be substituted with deuterium; at least one of $R^{1'}$, $R^{2'}$, and $R^{3'}$ is an aromatic group; m1' is 0 or 1; and m3' is 1,

(C) a ligand which is a nitrogen-containing aromatic compound with monodentate coordination to M, and

(D) a ligand which is a nitrogen-containing aromatic compound with bidentate coordination to M.

In formula (A), $R^1$, $R^2$, and $R^3$ are the same or different and are each a hydrogen atom, a deuterium atom, an alkyl group having 1 to 20 carbon atoms, an alkyloxy group having 1 to 20 carbon atoms, an aromatic group, or an aryloxy group, and these groups may be substituted with deuterium. At least one of $R^1$, $R^2$, and $R^3$, though, is an aromatic group or an aryloxy group. $R^1$, $R^2$, and $R^3$ are preferably hydrogen atoms, alkyl groups having 1 to 20 carbon atoms, alkyloxy groups having 1 to 20 carbon atoms, aromatic groups, aryloxy groups, or these groups substituted with deuterium. Alkyl groups having 1 to 20 carbon atoms, aromatic groups and the like are particularly preferable.

In formula (A), X is a phosphorus or sulfur atom.

In formula (A), m1 is 0 or 1.

In formula (A), when X is a phosphorus atom, m3 is 1, and when X is a sulfur atom, m3 is 0.

The following are examples of the ligand represented by formula (A). When X is a phosphorus atom, examples include triphenylphosphine, triphenylphosphine oxide, triphenyl phosphite, and the like. When X is a sulfur atom, examples include diphenyl sulfide, diphenyl sulfoxide, and the like.

In formula (B), $R^{1'}$, $R^{2'}$, and $R^{3'}$ are the same or different and are each a hydrogen atom, a deuterium atom, an alkyl group having 1 to 20 carbon atoms, an alkyloxy group having 1 to 20 carbon atoms, or an aromatic group, and these groups may be substituted with deuterium. At least one of $R^{1'}$, $R^{2'}$, and $R^{3'}$, though, is an aromatic group. $R^{1'}$, $R^{2'}$, and $R^{3'}$ are preferably hydrogen atoms, alkyl groups having 1 to 20 carbon atoms, alkyloxy groups having 1 to 20 carbon atoms, aromatic groups or these groups substituted with deuterium. Alkyl groups having 1 to 20 carbon atoms, aromatic groups, and the like are particularly preferable.

In formula (B), m1' is 0 or 1, and preferably 0.

In formula (B), m3' is 1.

Examples of the ligand represented by formula (B) include triphenylamine, diphenylamine, aniline, and the like. Triphenylamine, diphenylamine, and the like are particularly preferable as the ligand represented by formula (B).

Examples of the alkyl groups having 1 to 20 carbon atoms represented by $R^1$, $R^2$, $R^3$, $R^{1'}$, $R^{2'}$, and $R^{3'}$ include methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, icosyl groups and the like. The carbon number of the alkyl groups represented by $R^1$, $R^2$, $R^3$, $R^{1'}$, $R^{2'}$, and $R^{3'}$ is usually 1 to 20, preferably 1 to 8, and particularly preferably 1 to 4.

The alkyl groups having 1 to 20 carbon atoms represented by $R^1$, $R^2$, $R^3$, $R^{1'}$, $R^{2'}$, and $R^{3'}$ may be straight-chain or branched-chain.

Examples of the alkyloxy groups having 1 to 20 carbon atoms represented by $R^1$, $R^2$, $R^3$, $R^{1'}$, $R^{2'}$, and $R^{3'}$ include methoxy, ethoxy, propyloxy, pentyloxy groups and the like. The carbon number of the alkyloxy groups is usually 1 to 20, preferably 1 to 8, and particularly preferably 1 to 4.

Examples of the aromatic groups represented by $R^1$, $R^2$, $R^3$, $R^{1'}$, $R^{2'}$, and $R^{3'}$ include phenyl group; tolyl and other such phenyl groups having alkyl substituents having 1 to 3 carbon atoms; chlorophenyl and other such phenyl groups having halogen substituents (such as chlorine, fluorine, bromine, etc.); naphthyl group, and the like.

Examples of the aryloxy groups represented by $R^1$, $R^2$, and $R^3$ include phenoxy group; methylphenoxy and other such phenoxy groups having alkyl substituent(s) having 1 to 3 carbon atoms; chlorophenoxy and other phenoxy groups having halogen substituents (such as chlorine, fluorine, bromine etc.); the naphthyloxy group, and the like.

The various groups indicated by $R^1$, $R^2$, $R^3$, $R^{1'}$, $R^{2'}$, and $R^{3'}$ may also be substituted with deuterium.

In view of the luminescence intensity, at least one of $R^1$, $R^2$, and $R^3$ in the ligand represented by formula (A) is preferably an aromatic group. All of $R^1$, $R^2$, and $R^3$ are more preferably aromatic groups, and particularly preferably phenyl groups.

In view of the luminescence intensity, at least one of $R^{1'}$, $R^{2'}$, and $R^{3'}$ in the ligand represented by formula

(B) is preferably an aromatic group. All of $R^{1'}$, $R^{2'}$, and $R^{3'}$ are more preferably aromatic groups, and particularly preferable phenyl groups.

Examples of the ligand that is a nitrogen-containing aromatic compound with monodentate coordination to M include pyridine, pyrazine, pyrimidine, pyridazine, triazine and the like. Pyridine is preferable as the nitrogen-containing aromatic compound with monodentate coordination to M.

Examples of the ligand that is a nitrogen-containing aromatic compound with bidentate coordination to M include bipyridine (such as 2,2'-bipyridine), phenanthroline (such as 1,10-phenanthroline), and the like. 2,2'-bipyridine and 1,10-phenanthroline are preferable as the nitrogen-containing aromatic compound with bidentate coordination to M.

In terms of the luminescence intensity and chemical stability of the rare earth ternary complex represented by formula (1), a complex wherein Z is a ligand represented by formula (A) is preferable, and a complex wherein X in formula (A) is a phosphorus atom is more preferable.

The ligand represented by Z is preferably triphenylphosphine, triphenylphosphine oxide, diphenyl sulfide, diphenyl sulfoxide, pyridine, bipyridine, phenanthroline, or the like.

m2 is the coordination number of the ligand represented by Z in the complex represented by formula (1). m2 is an integer from 1 to 10 when Z is at least one ligand selected from the group consisting of (A) to (C), and is an integer from 1 to 5 when Z is at least one ligand selected from among (D).

When Z is at least one ligand selected from the group consisting of (A) to (C), m2 is preferably 2, 6, or 8, and particularly preferably 8.

When Z is at least one ligand selected from among (D), m2 is preferably 1, 3, or 4, and particularly preferably 4. Specific examples of the rare earth ternary complex represented by formula (1) include the following complexes. In the following formulas, x represents a number from 1 to 22.

- a rare earth ternary complex of $[(C_xF_{2x+1}SO_2)_2N]_{n2}M$ and triphenylphosphine,
- a rare earth ternary complex of $[(C_xF_{2x+1}SO_2)_2N]_{n2}M$ and triphenylphosphine oxide,
- a rare earth ternary complex of $[(C_xF_{2x+1}SO_2)_2N]_{n2}M$ and diphenyl sulfide,
- a rare earth ternary complex of $[(C_xF_{2x+1}SO_2)_2N]_{n2}M$ and diphenyl sulfoxide,
- a rare earth ternary complex of $[(C_xF_{2x+1}SO_2)_2N]_{n2}M$ and diphenylamine,
- a rare earth ternary complex of $[(C_xF_{2x+1}SO_2)_2N]_{n2}M$ and triphenylamine,
- a rare earth ternary complex of $[(C_xF_{2x+1}SO_2)_2N]_{n2}M$ and aniline,
- a rare earth ternary complex of $[(C_xF_{2x+1}SO_2)_2N]_{n2}M$ and pyridine,
- a rare earth ternary complex of $[(C_xF_{2x+1}SO_2N]_{n2}M$ and bipyridine,
- a rare earth ternary complex of $[(C_xF_{2x+1}SO_2)_2N]_{n2}M$ and phenanthroline,
- a rare earth ternary complex of $[(C_xCl_{2x+1}SO_2)_2N]_{n2}M$ and triphenylphosphine,
- a rare earth ternary complex of $[(C_xCl_{2x+1}SO_2)_2N]_{n2}M$ and triphenylphosphine oxide,
- a rare earth ternary complex of $[(C_xCl_{2x+1}SO_2)_2N]_{n2}M$ and diphenyl sulfide,
- a rare earth ternary complex of $[(C_xCl_{2x+1}SO_2)_2N]_{n2}N$ and diphenyl sulfoxide,
- a rare earth ternary complex of $[(C_xCl_{2x+1}SO_2)_2N]_{n2}M$ and diphenylamine,
- a rare earth ternary complex of $[(C_xCl_{2x+1}SO_2)_2N]_{n2}M$ and triphenylamine,
- a rare earth ternary complex of $[(C_xCl_{2x+1}SO_2)_2N]_{n2}M$ and aniline,
- a rare earth ternary complex of $[(C_xCl_{2x+1}SO_2)_2N]_{n2}M$ and pyridine,
- a rare earth ternary complex of $[(C_xCl_{2x+1}SO_2)_2N]_{n2}M$ and bipyridine,
- a rare earth ternary complex of $[(C_xCl_{2x+1}SO_2)_2N]_{n2}M$ and phenanthroline,
- a rare earth ternary complex of $[(C_6F_5SO_2)_2N]_{n2}M$ and triphenylphosphine,
- a rare earth ternary complex of $[(C_6F_5SO_2)_2N]_{n2}M$ and triphenylphosphine oxide,
- a rare earth ternary complex of $[(C_6F_5SO_2)_2N]_{n2}M$ and diphenyl sulfide,
- a rare earth ternary complex of $[(C_6F_5SO_2)_2N]_{n2}M$ and diphenyl sulfoxide,
- a rare earth ternary complex of $[(C_6F_5SO_2)N]_{n2}M$ and diphenylamine,
- a rare earth ternary complex of $[(C_6F_5SO_2)_2N]_{n2}M$ and triphenylamine,
- a rare earth ternary complex of $[(C_6F_5SO_2)_2N]_{n2}M$ and aniline,
- a rare earth ternary complex of $[(C_6F_5SO_2)_2N]_{n2}M$ and pyridine,
- a rare earth ternary complex of $[(C_6F_5SO_2)_2N]_{n2}M$ and bipyridine, and
- a rare earth ternary complex of $[(C_6F_5SO_2)_2N]_{n2}M$ and phenanthroline.

The following are further examples of the complexes.

* The complexes listed below, having a $[(C_xF_{Zx-1}SO_2)_2N]_{n2}M$ structure (wherein $C_xF_{2x-1}$ is a perfluoroalkenyl group, and x is an integer from 2 to 22)

- a rare earth ternary complex of $[(C_xF_{2x-1}SO_2)_2N]_{n2}M$ (x = 2 to 22) and triphenylphosphine
- a rare earth ternary complex of $[(C_xF_{2x-1}SO_2)_2N]_{n2}M$ (x = 2 to 22) and triphenylphosphine oxide
- a rare earth ternary complex of $[(C_xF_{2x-1}SO_2)_2N]_{n2}M$ (x = 2 to 22) and diphenyl sulfide
- a rare earth ternary complex of $[(C_xF_{2x-1}SO_2)_2N]_{n2}M$ (x = 2 to 22) and diphenyl sulfoxide
- a rare earth ternary complex of $[(C_xF_{2x-1}SO_2)_2N]_{n2}M$ (x = 2 to 22) and diphenylamine
- a rare earth ternary complex of $[(C_xF_{2x-1}SO_2)_2N]_{n2}M$ (x = 2 to 22) and triphenylamine
- a rare earth ternary complex of $[(C_xF_{2x-1}SO_2)_2N]_{n2}M$ (x = 2 to 22) and aniline
- a rare earth ternary complex of $((C_xF_{2x-1}SO_2)_2N]_{n2}M$ (x = 2 to 22) and pyridine
- a rare earth ternary complex of $[(C_xP_{2x-1}SO_2)_2N]_{n2}M$ (x = 2 to 22) and bipyridine
- a rare earth ternary complex of $[(C_xF_{2x-1}SO_2)_2N]_{n2}M$ (x = 2 to 22) and phenanthroline

* The complexes listed below, having a $(C_xCl_{2x+1}SO_2)_2N]_{n2}M$ structure (wherein $C_xCl_{2x-1}$ is a perchloroalkenyl group, and x is an integer from 2 to 22)

- a rare earth ternary complex of $[(C_xCl_{2x-1}SO_2)_2N]_{n2}M$ (x = 2 to 22) and triphenylphosphine
- a rare earth ternary complex of $[(C_xCl_{2x-1}SO_2)_2N]_{n2}M$ (x = 2 to 22) and triphenylphosphine oxide
- a rare earth ternary complex of $[(C_xCl_{2x-1}SO_2)_2N]_{n2}M$ (x = 2 to 22) and diphenyl sulfide
- a rare earth ternary complex of $(C_xCl_{2x-1}SO_2)_2N]_{n2}M$ (x = 2 to 22) and diphenyl sulfoxide
- a rare earth ternary complex of $[(C_xCl_{2x-1}SO_2)_2N]_{n2}M$ (x = 2 to 22) and diphenylamine
- a rare earth ternary complex of $[(C_xCl_{2x-1}SO_2)_2N]_{n2}M$ (x = 2 to 22) and triphenylamine
- a rare earth ternary complex of $[(C_xCl_{2x-1}SO_2)_2N]_{n2}M$ (x = 2 to 22) and aniline
- a rare earth ternary complex of $[(C_xCl_{2x-1}SO_2)_2N]_{n2}M$ (x = 2 to 22) and pyridine
- a rare earth ternary complex of $[(C_xCl_{2x-1}SO_2)_2N]_{n2}M$ (x = 2 to 22) and bipyridine
- a rare earth ternary complex of $[(C_xCl_{2x-1}SO_2)_2N]_{n2}M$ (x = 2 to 22) and phenanthroline

* The complexes listed below, having a $[(CF_3\text{-}C_6F_4SO_2)_2N]_{n2}M$ structure

- a rare earth ternary complex of $[(CF_3\text{-}C_6F_4SO_2)_2N]_{n2}M$ and triphenylphosphine
- a rare earth ternary complex of $[(CF_3\text{-}C_6F_4SO_2)_2N]_{n2}M$ and triphenylphosphine oxide
- a rare earth ternary complex of $[(CF_3\text{-}C_6F_4SO)_2N]_{n2}M$ and diphenyl sulfide
- a rare earth ternary complex of $[(CF_3\text{-}C_6F_4SO_2)_2N]_{n2}M$ and diphenyl sulfoxide
- a rare earth ternary complex of $[(CF_3\text{-}C_6F_4SO_2)_2N]_{n2}M$ and diphenylamine
- a rare earth ternary complex of $[(CF_3\text{-}C_6F_4SO_2)_2N]_{n2}M$ and triphenylamine
- a rare earth ternary complex of $[(CF_3\text{-}C_6F_4SO_2)_2N]_{n2}M$ and aniline
- a rare earth ternary complex of $[(CF_3\text{-}C_6F_4SO_2)_2N]_{n2}M$ and pyridine
- a rare earth ternary complex of $[(CF_3\text{-}C_6F_4SO_2)_2N]_{n2}M$ and bipyridine
- a rare earth ternary complex of $[(CF_3\text{-}C_6F_4SO_2)_2N]_{n2}M$ and phenanthroline

* The complexes listed below, having a $[(CCl_3\text{-}C_6Cl_4SO_2)_2N]_{n2}M]$structure

- a rare earth ternary complex of $[(CCl_3\text{-}C_6Cl_4SO_2)_2N]_{n2}M$ and triphenylphosphine
- a rare earth ternary complex of $[(CCl_3\text{-}C_6Cl_4SO_2)_2N]_{n2}M$ and triphenylphosphine oxide
- a rare earth ternary complex of $[(CCl_3\text{-}C_6Cl_4SO_2)_2N]_{n2}M$ and diphenyl sulfide
- a rare earth ternary complex of $[(CCl_3\text{-}C_6Cl_4SO_2)_2N]_{n2}M$ and diphenyl sulfoxide
- a rare earth ternary complex of $[(CCl_3\text{-}C_6Cl_4SO_2)_2N]_{n2}M$ and diphenylamine
- a rare earth ternary complex of $[(CCl_3\text{-}C_6Cl_4SO_2)_2N]_{n2}M$ and triphenylamine
- a rare earth ternary complex of $[(CCl_3\text{-}C_6Cl_4SO_2)_2N]_{n2}M$ and aniline
- a rare earth ternary complex of $[(CCl_3\text{-}C_6Cl_4SO_2)_2N]_{n2}M$ and pyridine
- a rare earth ternary complex of $[(CCl_3\text{-}C_6Cl_4SO_2)_2N]_{n2}M$ and bipyridine
- a rare earth ternary complex of $[(CCl_3\text{-}C_6Cl_4SO_2)_2N]_{n2}M$ and phenanthroline

* The complexes listed below, having a $[(C_xF_{2x+1}SO_2)\ (C_yF_{2y+1}SO_2)N]_{n2}M$ structure (wherein x and y are different from each other, and are each an integer from 1 to 22)

- a rare earth ternary complex of $[(C_xF_{2+1}SO_2)\ (C_yF_{2y+1}SO_2)N]_{n2}M$ (x, y = 1 to 22) and triphenylphosphine
- a rare earth ternary complex of $(C_xF_{2x+1}SO_2)\ (C_yF_{2y+1}SO_2)N]_{n2}M$ (x, y = 1 to 22) and triphenylphosphine oxide
- a rare earth ternary complex of $[(C_xF_{2x+1}SO_2)\ (C_yF_{2y+1}SO_2)N]_{n2}M$ (x, y = 1 to 22) and diphenyl sulfide
- a rare earth ternary complex of $[(C_xF_{2x+1}SO_2)\ (C_yF_{2y+1}SO_2)N]_{n2}M$ (x, y = 1 to 22) and diphenyl sulfoxide

- a rare earth ternary complex of $[(C_xF_{2x+1}SO_2)\ (C_yF_{2y+1}SO_2)N]_{n2}M$ (x, y = 1 to 22) and diphenylamine
- a rare earth ternary complex of $[(C_xF_{2x+1}SO_2)\ (C_yF_{2y+1}SO_2)N]_{n2}M$ (x, y = 1 to 22) and triphenylamine
- a rare earth ternary complex of $[(C_xF_{2x+1}SO_2)\ (C_yF_{2y+1}SO_2)N]_{n2}M$ (x, y = 1 to 22) and aniline
- a rare earth ternary complex of $[(C_xF_{2x+1}SO_2)\ (C_yF_{2y+1}SO_2)N]_{n2}M$ (x, y = 1 to 22) and pyridine
- a rare earth ternary complex of $[(C_xF_{2x+1}SO_2)\ (C_yF_{2y+1}SO_2)N]_{n2}M$ (x, y = 1 to 22) and bipyridine
- a rare earth ternary complex of $[(C_xF_{2x+1}SO_2)\ (C_yF_{2y+1}SO_2)N]_{n2}M$ (x, y = 1 to 22) and phenanthroline

* The complexes listed below, having a $[(C_xCl_{2x+1}SO_2)\ (C_yCl_{2y+1}SO_2)N]_{n2}M$ structure (wherein x and y are different from each other, and are each an integer from 1 to 22)

  - a rare earth ternary complex of $[(C_xCl_{2x+1}SO_2)\ (C_yCl_{2y+1}SO_2)N]_{n2}M$ (x, y = 1 to 22) and triphenylphosphine
  - a rare earth ternary complex of $[(C_xCl_{2x+1}SO_2)\ (C_yCl_{2y+1}SO_2)N]_{n2}M$ (x, y = 1 to 22) and triphenylphosphine oxide
  - a rare earth ternary complex of $[(C_xCl_{2x+1}SO_2)\ (C_yCl_{2y+1}SO_2)N]_{n2}M$ (x, y = 1 to 22) and diphenyl sulfide
  - a rare earth ternary complex of $[(C_xCl_{2x+1}SO_2)\ (C_yCl_{2y+1}SO_2)N]_{n2}M$ (x, y = 1 to 22) and diphenyl sulfoxide
  - a rare earth ternary complex of $[(C_xCl_{2x+1}SO_2)\ (C_yCl_{2y+1}SO_2)N]_{n2}M$ (x, y = 1 to 22) and diphenylamine
  - a rare earth ternary complex of $[(C_xCl_{2x+1}SO_2)\ (C_yCl_{2y+1}SO_2)N]_{n2}M$ (x, y = 1 to 22) and triphenylamine
  - a rare earth ternary complex of $[(C_xCl_{2x+1}SO_2)\ (C_yCl_{2y+1}SO_2)N]_{n2}M$ (x, y = 1 to 22) and aniline
  - a rare earth ternary complex of $[(C_xCl_{2x+1}SO_2)\ (C_yCl_{2y+1}SO_2)N]_{n2}M$ (x, y = 1 to 22) and pyridine
  - a rare earth ternary complex of $[(C_xCl_{2x+1}SO_2)\ (C_yCl_{2y+1}SO_2)N]_{n2}M$ (x, y = 1 to 22) and bipyridine
  - a rare earth ternary complex of $[(C_xCl_{2x+1}SO_2)\ (C_yCl_{2y+1}SO_2)N]_{n2}M$ (x, y = 1 to 22) and phenanthroline

* The complexes listed below, having a $[(C_xF_{2x+1}SO_2)\ (C_yCl_{2y+1}SO_2)N]_{n2}M$ structure (wherein x and y are the same or different, and are each an integer from 1 to 22)

  - a rare earth ternary complex of $[(C_xF_{2x+1}SO_2)\ (C_yCl_{2y+1}SO_2N]_{n2}M$ (x, y = 1 to 22) and triphenylphosphine
  - a rare earth ternary complex of $[(C_xF_{2x+1}SO_2)\ (C_yCl_{2y+1}SO_2)N]_{n2}M$ (x, y=1 to 22) and triphenylphosphine oxide
  - a rare earth ternary complex of $[(C_xF_{2x+1}SO_2)\ (C_yCl_{2y+1}SO_2)N]_{n2}M$ (x, y = 1 to 22) and diphenyl sulfide
  - a rare earth ternary complex of $[(C_xF_{2x+1}SO_2)\ (C_yCl_{2y+1}SO_2)N]_{n2}M$ (x, y = 1 to 22) and diphenyl sulfoxide
  - a rare earth ternary complex of $[(C_xF_{2x+1}SO_2)\ (C_yCl_{2y+1}SO_2)N]_{n2}M$ (x, y = 1 to 22) and diphenylamine
  - a rare earth ternary complex of $[(C_xF_{2x+1}SO_2)\ (C_yCl_{2y+1}SO_2)N]_{n2}M$ (x, y = 1 to 22) and triphenylamine
  - a rare earth ternary complex of $[(C_xF_{2x+1}SO_2)\ (C_yCl_{2y+1}SO_2)N]_{n2}M$ (x, y = 1 to 22) and aniline
  - a rare earth ternary complex of $[(C_xF_{2x+1}SO_2)\ (C_yCl_{2y+1}SO_2)N]_{n2}M$ (x, y = 1 to 22) and pyridine
  - a rare earth ternary complex of $[(C_xF_{2x+1}SO_2)\ (C_yCl_{2y+1}SO_2)N]_{n2}M$ (x, y = 1 to 22) and bipyridine
  - a rare earth ternary complex of $[(C_xF_{2x+1}SO_2)\ (C_yCl_{2y+1}SO_2)N)_{n2}M$ (x, y = 1 to 22) and phenanthroline

* The complexes listed below, having a $[(C_xF_{2x-1}SO_2)(C_yF_{2y-1}SO_2)N]_{n2}M$ structure (wherein $C_xF_{2x-1}$ and $C_yF_{2y-1}$ are each a perfluoroalkenyl group, x and y are different from each other, and x and y are each an integer from 2 to 22)

  - a rare earth ternary complex of $[(C_xF_{2x-1}SO_2)\ (C_yF_{2y-1}SO_2)N]_{n2}M$ (x, y = 2 to 22) and triphenylphosphine
  - a rare earth ternary complex of $[(C_xF_{2x-1}SO_2)\ (C_yF_{2y-1}SO_2)N]_{n2}M$ (x, y = 2 to 22) and triphenylphosphine oxide
  - a rare earth ternary complex of $[(C_xF_{2x-1}SO_2)\ (C_yF_{2y-1}SO_2)N]_{n2}M$ (x, y = 2 to 22) and diphenyl ulfide
  - a rare earth ternary complex of $[(C_xF_{2x-1}SO_2)\ (CyF_{2y-1}SO_2)N]_{n2}M$ (x, y = 2 to 22) and diphenyl sulfoxide
  - a rare earth ternary complex of $[(C_xF_{2x-1}SO_2)(C_yF_{2y-1}SO_2)N]_{n2}M$ (x, y = 2 to 22) and diphenylamine
  - a rare earth ternary complex of $[(C_xF_{2x-1}SO_2)\ (C_yF_{2y-1}SO_2)N]_{n2}M$ (x, y = 2 to 22) and triphenylamine
  - a rare earth ternary complex of $[(C_xF_{2x-1}SO_2)\ (C_yF_{2y-1}SO_2)N]_{n2}M$ (x, y = 2 to 22) and aniline
  - a rare earth ternary complex of $[(C_xF_{2x-1}SO_2)\ (C_yF_{2y-1}SO_2)N]_{n2}M$ (x, y = 2 to 22) and pyridine
  - a rare earth ternary complex of $[(C_xF_{2x-1}SO_2)\ (C_yF_{2y-1}SO_2N]_{n2}M$ (x, y = 2 to 22) and bipyridine
  - a rare earth ternary complex of $[(C_xF_{2x-1}SO_2)\ (C_yF_{2y-1}SO_2)N]_{n2}M$ (x, y = 2 to 22) and phenanthroline

* The complexes listed below, having a $[(C_xCl_{2x-1}SO_2)\ (CyCl_{2y-1}SO_2)N]_{n2}M$ structure (wherein $C_xCl_{2x-1}$ and $C_yCl_{2y-1}$ are each a perchloroalkenyl group, and x and y are different from each other, and x and y are each an integer from 2 to 22)

  - a rare earth ternary complex of $[(C_xCl_{2x-1}SO_2)\ (C_yCl_{2y-1}SO_2)N]_{n2}M$ (x, y = 2 to 22) and triphenylphosphine
  - a rare earth ternary complex of $[(C_xCl_{2x-1}SO_2)\ (C_yCl_{2y-1}SO_2)N]_{n2}M$ (x, y = 2 to 22) and triphenylphosphine oxide
  - a rare earth ternary complex of $[(C_xCl_{2x-1}SO_2)\ (C_yCl_{2y-1}SO_2)N]_{n2}M$ (x, y = 2 to 22) and diphenyl sulfide
  - a rare earth ternary complex of $[(C_xCl_{2x-1}SO_2)\ (C_yCl_{2y-1}SO_2)N]_{n2}M$ (x, y = 2 to 22) and diphenyl sulfoxide

- a rare earth ternary complex of $[(C_xCl_{2x-1}SO_2)\ (C_yCl_{2y-1}SO_2)N]_{n2}M$ (x, y = 2 to 22) and diphenylamine
- a rare earth ternary complex of $[(C_xCl_{2x-1}SO_2)\ (C_yCl_{2y-1}SO_2)N]_{n2}M$ (x, y = 2 to 22) and triphenylamine
- a rare earth ternary complex of $[(C_xCl_{2x-1}SO_2)\ (C_yCl_{2y-1}SO_2)N]_{n2}M$ (x, y = 2 to 22) and aniline
- a rare earth ternary complex of $[(C_xCl_{2x-1}SO_2)\ (C_yCl_{2y-1}SO_2)N]_{n2}M$ (x, y = 2 to 22) and pyridine
- a rare earth ternary complex of $[(C_xCl_{2x-1}SO_2)\ (C_yCl_{2y-1}SO_2)N]_{n2}M$ (x, y = 2 to 22) and bipyridine
- a rare earth ternary complex of $[(C_xCl_{2x-1}SO_2)\ (C_yCl_{2y-1}SO_2)N]_{n2}M$ (x, y = 2 to 22) and phenanthroline

* The complexes listed below, having a $[(C_xF_{2x-1}SO_2)\ (C_yCl_{2y-1}SO_2)N]_{n2}M$ structure (wherein $C_xF_{2x-1}$ is a-perfluoroalkenyl group and $C_xCl_{2x-1}$ is a perchloroalkenyl group, and x and y are the same or different and are each an integer from 2 to 22)

- a rare earth ternary complex of $[(C_xF_{2x-1}SO_2)\ (C_yCl_{2y-1}SO_2)N]_{n2}M$ (x, y = 2 to 22) and triphenylphosphine
- a rare earth ternary complex of $[(C_xF_{2x-1}SO_2)\ (C_yCl_{2y-1}SO_2)N]_{n2}M$ (x, y = 2 to 22) and triphenylphosphine oxide
- a rare earth ternary complex of $[(C_xF_{2x-1}SO_2)\ (C_yCl_{2y-1}SO_2)N]_{n2}M$ (x, y = 2 to 22) and diphenyl sulfide
- a rare earth ternary complex of $[(C_xF_{2x-1}SO_2)\ (C_yCl_{2y-1}SO_2)N]_{n2}M$ (x, y = 2 to 22) and diphenyl sulfoxide
- a rare earth ternary complex of $[(C_xF_{2x-1}SO_2)\ (C_yCl_{2y-1}SO_2)N]_{n2}M$ (x, y = 2 to 22) and diphenylamine
- a rare earth ternary complex of $[(C_xF_{2x-1}SO_2)\ (C_yCl_{2y-1}SO_2)N]_{n2}M$ (x, y = 2 to 22) and triphenylamine
- a rare earth ternary complex of $[(C_xF_{2x+1}SO_2)\ (C_yCl_{2y-1}SO_2)N]_{n2}M$ (x, y = 2 to 22) and aniline
- a rare earth ternary complex of $(C_xF_{2x-1}SO_2)\ (C_yCl_{2y-1}SO_2)N]_{n2}M$ (x, y = 2 to 22) and pyridine
- a rare earth ternary complex of $[(C_xF_{2x-1}SO_2)\ (C_yCl_{2y-1}SO_2)N]_{n2}M$ (x, y = 2 to 22) and bipyridine
- a rare earth ternary complex of $[(C_xF_{2x-1}SO_2)\ (C_yCl_{2y-1}SO_2)N]_{n2}M$ (x, y = 2 to 22) and phenanthroline

* The complexes listed below, having a $[(C_6F_5SO_2)\ (C_xF_{2x+1}SO_2)N]_{n2}M$ structure (wherein x is an integer from 1 to 22)

- a rare earth ternary complex of $[(C_6F_5SO_2)\ (C_xF_{2x+1}SO_2)N]_{n2}M$ (x = 1 to 22) and triphenylphosphine
- a rare earth ternary complex of $[(C_6F_5SO_2)\ (C_xF_{2x+1}SO_2)N]_{n2}M$ (x = 1 to 22) and triphenylphosphine oxide
- a rare earth ternary complex of $[(C_6F_5SO_2)\ (C_xF_{2x+1}SO_2)N]_{n2}N$ (x = 1 to 22) and diphenyl sulfide
- a rare earth ternary complex of $[(C_6F_5SO_2)\ (C_xF_{2x+1}SO_2)N]_{n2}M$ (x = 1 to 22) and diphenyl sulfoxide
- a rare earth ternary complex of $[(C_6F_5SO_2)\ (C_xF_{2x+1}SO_2)N]_{n2}M$ (x = 1 to 22) and diphenylamine
- a rare earth ternary complex of $[(C_6F_5SO_2)\ (C_xF_{2x+1}SO_2)N]_{n2}M$ (x = 1 to 22) and triphenylamine
- a rare earth ternary complex of $[(C_6F_5SO_2)\ (C_xF_{2x+1}SO_2)N]_{n2}M$ (x = 1 to 22) and aniline
- a rare earth ternary complex of $[(C_6F_5SO_2)\ (C_xF_{2x+1}SO_2)N)_{n2}M$ (x = 1 to 22) and pyridine
- a rare earth ternary complex of $[(C_6F_5SO_2)\ (C_xF_{2x+1}SO_2)N]_{n2}M$ (x = 1 to 22) and bipyridine
- a rare earth ternary complex of $[(C_6F_5SO_2)\ (C_xF_{2x+1}SO_2)N]_{n2}M$ (x = 1 to 22) and phenanthroline

* The complexes listed below, having a $[(CF_3\text{-}C_6F_4SO_2)\ (C_xF_{2x+1}SO_2)N]_{n2}M$ structure (wherein x is an integer from 1 to 22)

- a rare earth ternary complex of $[(CF_3\text{-}C_6F_4SO_2)\ (C_xF_{2x+1}SO_2)N]_{n2}M$ (x = 1 to 22) and triphenylphosphine
- a rare earth ternary complex of $[(CF_3\text{-}C_6F_4SO_2)\ (C_xF_{2x+1}SO_2)N]_{n2}M$ (x = 1 to 22) and triphenylphosphine oxide
- a rare earth ternary complex of $[(CF_3\text{-}C_6F_4SO_2)\ (C_xF_{2x+1}SO_2)N]_{n2}N$ (x = 1 to 22) and diphenyl sulfide
- a rare earth ternary complex of $[(CF_3\text{-}C_6F_4SO_2)\ (C_xF_{2x+1}SO_2)N]_{n2}N$ (x = 1 to 22) and diphenyl sulfoxide
- a rare earth ternary complex of $[(CF_3\text{-}C_6F_4SO_2)\ (C_xF_{2x+1}SO_2)N]_{n2}M$ (x = 1 to 22) and diphenylamine
- a rare earth ternary complex of $[(CF_3\text{-}C_6F_4SO_2)\ (C_xF_{2x+1}SO_2)N]_{n2}M$ (x = 1 to 22) and triphenylamine
- a rare earth ternary complex of $[(CF_3\text{-}C_6F_4SO_2)\ (C_xF_{2x+1}SO_2)N]_{n2}M$ (x = 1 to 22) and aniline
- a rare earth ternary complex of $[(CF_3\text{-}C_6F_4SO_2)\ (C_xF_{2x+1}SO_2)N]_{n2}M$ (x = 1 to 22) and pyridine
- a rare earth ternary complex of $[(CF_3\text{-}C_6F_4SO_2)\ (C_xF_{2x+1}SO_2)N]_{n2}M$ (x = 1 to 22) and bipyridine
- a rare earth ternary complex of $[(CF_3\text{-}C_6F_4SO_2)\ (C_xF_{2x+1}SO_2)N]_{n2}M$ (x = 1 to 22) and phenanthroline

* The complexes listed below, having a $[(CCl_3\text{-}C_6Cl_4SO_2)\ (C_xF_{2x+1}SO_2)N]_{n2}M$ structure (wherein x is an integer from 1 to 22)

- a rare earth ternary complex of $[(CCl_3\text{-}C_6Cl_4SO_2)\ (C_xF_{2x+1}SO_2)N]_{n2}M$ (x = 1 to 22) and triphenylphosphine
- a rare earth ternary complex of $[(CCl_3\text{-}C_6Cl_4SO_2)\ (C_xF_{2x+1}SO_2)N]_{n2}M$ (x = 1 to 22) and triphenylphosphine oxide
- a rare earth ternary complex of $[(CCl_3\text{-}C_6Cl_4SO_2)\ (C_xF_{2x+1}SO_2)N]_{n2}M$ (x = 1 to 22) and diphenyl sulfide
- a rare earth ternary complex of $[(CCl_3\text{-}C_6Cl_4SO_2)\ (C_xF_{2x+1}SO_2)N]_{n2}M$ (x = 1 to 22) and diphenyl sulfoxide

- a rare earth ternary complex of $[(CCl_3\text{-}C_6Cl_4SO_2)\ (C_xF_{2x+1}SO_2)N]_{n2}M$ (x = 1 to 22) and diphenylamine
- a rare earth ternary complex of $[(CCl_3\text{-}C_6Cl_4SO_2)\ (C_xF_{2x+1}SO_2)N]_{n2}M$ (x = 1 to 22) and triphenylamine
- a rare earth ternary complex of $[(CCl_3\text{-}C_6Cl_4SO_2)\ (C_xF_{2x+1}SO_2)N]_{n2}M$ (x = 1 to 22) and aniline
- a rare earth ternary complex of $[(CCl_3\text{-}C_6Cl_4SO_2)\ (C_xF_{2x+1}SO_2)N]_{n2}M$ (x = 1 to 22) and pyridine
- a rare earth ternary complex of $[(CCl_3\text{-}C_6Cl_4SO_2)\ (C_xF_{2x+1}SO_2)N]_{n2}M$ (x = 1 to 22) and bipyridine
- a rare earth ternary complex of $[(CCl_3\text{-}C_6Cl_4SO_2)\ (C_xF_{2x+1}SO_2)N]_{n2}M$ (x = 1 to 22) and phenanthroline

* The complexes listed below, having a $[(C_6Cl_5SO_2)\ (C_xF_{2x+1}SO_2)N]_{n2}M$ structure (wherein x is an integer from 1 to 22)

  - a rare earth ternary complex of $[(C_6Cl_5SO_2)\ (C_xF_{2x+1}SO_2)N]_{n2}M$ (x = 1 to 22) and triphenylphosphine
  - a rare earth ternary complex of $[(C_6Cl_5SO_2)\ (C_xF_{2x+1}SO_2)N]_{n2}M$ (x = 1 to 22) and triphenylphosphine oxide
  - a rare earth ternary complex of $[(C_6Cl_5SO_2)\ (C_xF_{2x+1}SO_2)N]_{n2}M$ (x = 1 to 22) and diphenyl sulfide
  - a rare earth ternary complex of $[(C_6Cl_5SO_2)\ (C_xF_{2x+1}SO_2)N]_{n2}M$ (x = 1 to 22) and diphenyl sulfoxide
  - a rare earth ternary complex of $[(C_6Cl_5SO_2)\ (C_xF_{2x+1}SO_2)N]_{n2}M$ (x = 1 to 22) and diphenylamine
  - a rare earth ternary complex of $[(C_6Cl_5SO_2)\ (C_xF_{2x+1}SO_2)N]_{n2}M$ (x = 1 to 22) and triphenylamine
  - a rare earth ternary complex of $[(C_6Cl_5SO_2)\ (C_xF_{2x+1}SO_2)N]_{n2}M$ (x = 1 to 22) and aniline
  - a rare earth ternary complex of $[(C_6Cl_5SO_2)\ (C_xF_{2x+1}SO_2)N]_{n2}M$ (x = 1 to 22) and pyridine
  - a rare earth ternary complex of $[(C_6Cl_5SO_2)\ (C_xF_{2x+1}SO_2)N]_{n2}M$ (x = 1 to 22) and bipyridine
  - a rare earth ternary complex of $[(C_6Cl_5SO_2)\ (C_xF_{2x+1}SO_2)N]_{n2}M$ (x = 1 to 22) and phenanthroline

* The complexes listed below, having a $[(C_6F_5SO_2)\ (C_xCl_{2x+1}SO_2)N]_{n2}M$ structure (wherein x is an integer from 1 to 22)

  - a rare earth ternary complex of $[(C_6F_5SO_2)\ (C_xCl_{2x+1}SO_2)N]_{n2}M$ (x = 1 to 22) and triphenylphosphine
  - a rare earth ternary complex of $[(C_6F_5SO_2)\ (C_xCl_{2x+1}SO_2)N]_{n2}M$ (x = 1 to 22) and triphenylphosphine oxide
  - a rare earth ternary complex of $[(C_6F_5SO_2)\ (C_xCl_{2+1}SO_2)N]\ _{n2}M$ (x = 1 to 22) and diphenyl sulfide
  - a rare earth ternary complex of $[(C_6F_5SO_2)\ (C_xCl_{2x+1}SO_2)N]_{n2}M$ (x = 1 to 22) and diphenyl sulfoxide
  - a rare earth ternary complex of $[(C_6F_5SO_2)\ (C_xCl_{2x+1}SO_2)N]_{n2}M$ (x = 1 to 22) and diphenylamine
  - a rare earth ternary complex of $[(C_6F_5SO_2)\ (C_xCl_{2x+1}SO_2)N]_{n2}M$ (x = 1 to 22) and triphenylamine
  - a rare earth ternary complex of $[(C_6F_5SO_2)\ (C_xCl_{2x+1}SO_2)N]_{n2}M$ (x = 1 to 22) and aniline
  - a rare earth ternary complex of $[(C_6F_5SO_2)\ (C_xCl_{2x+1}SO_2)N]_{n2}M$ (x = 1 to 22) and pyridine
  - a rare earth ternary complex of $(C_6F_5SO_2)\ (C_xCl_{2x+1}SO_2)N]_{n2}M$ (x = 1 to 22) and bipyridine
  - a rare earth ternary complex of $[(C_6F_5SO_2)\ (C_xCl_{2x+1}SO_2)N]_{n2}M$ (x = 1 to 22) and phenanthroline

* The complexes listed below, having a $[(C_6Cl_5SO_2)\ (C_xCl_{2x+1}SO_2)N]_{n2}M$ structure (wherein x is an integer from 1 to 22)

  - a rare earth ternary complex of $[(C_6Cl_5SO_2)\ (C_xCl_{2x+1}SO_2)N]_{n2}M$ (x = 1 to 22) and triphenylphosphine
  - a rare earth ternary complex of $[(C_6Cl_5SO_2)\ (C_xCl_{2x+1}SO_2)N]_{n2}M$ (x = 1 to 22 ) and triphenylphosphine oxide
  - a rare earth ternary complex of $[(C_6Cl_5SO_2)\ (C_xCl_{2x+1}SO_2)N]_{n2}M$ (x = 1 to 22) and diphenyl sulfide
  - a rare earth ternary complex of $[(C_6Cl_5SO_2)\ (C_xCl_{2x+1}SO_2)N]_{n2}N$ (x = 1 to 22) and diphenyl sulfoxide
  - a rare earth ternary complex of $[(C_6Cl_5SO_2)\ (C_xCl_{2x+1}SO_2)N]_{n2}M$ (x = 1 to 22) and diphenylamine
  - a rare earth ternary complex of $[(C_6Cl_5SO_2)\ (C_xCl_{2x+1}SO_2)N]_{n2}M$ (x = 1 to 22) and triphenylamine
  - a rare earth ternary complex of $[(C_6Cl_5SO_2)\ (C_xCl_{2x+1}SO_2)N]_{n2}M$ (x = 1 to 22) and aniline
  - a rare earth ternary complex of $[(C_6Cl_5SO_2)\ (C_xCl_{2x+1}SO_2)N]_{n2}M$ (x = 1 to 22) and pyridine
  - a rare earth ternary complex of $[(C_6Cl_5SO_2)\ (C_xCl_{2x+1}SO_2)N]_{n2}M$ (x = 1 to 22) and bipyridine
  - a rare earth ternary complex of $[(C_6Cl_5SO_2)\ (C_xCl_{2x+1}SO_2)N]_{n2}M$ (x = 1 to 22) and phenanthroline

Examples of preferable complexes include those listed below, in which x in the following formulas is an integer from 1 to 10.

- a rare earth ternary complex of $[(C_xF_{2x+1}SO_2)_2N]_{n2}M$ and triphenylphosphine
- a rare earth ternary complex of $[(C_xF_{2x+1}SO_2)_2N]_{n2}M$ and triphenylphosphine oxide
- a rare earth ternary complex of $[(C_xF_{2x+1}SO_2)_2N]_{n2}M$ and diphenyl sulfide
- a rare earth ternary complex of $[(C_xF_{2x+1}SO_2)_2N]_{n2}M$ and diphenyl sulfoxide
- a rare earth ternary complex of $[(C_xF_{2x+1}SO_2)_2N]_{n2}M$ and diphenylamine
- a rare earth ternary complex of $[(C_xF_{2x+1}SO_2)_2N]_{n2}M$ and triphenylamine

- a rare earth ternary complex of $[(C_xF_{2x+1}SO_2)_2N]_{n2}M$ and aniline
- a rare earth ternary complex of $[(C_xF_{2x+1}SO_2)_2N]_{n2}M$ and pyridine
- a rare earth ternary complex of $[(C_xF_{2x+1}SO_2)_2N]_{n2}M$ and bipyridine
- a rare earth ternary complex of $[(C_xF_{2x+1}SO_2)_2N]_{n2}M$ and phenanthroline

Further examples of preferable complexes include those listed below.

* The complexes listed below, having a $[(CCl_3\text{-}C_6Cl_4SO_2)_2N]_{n2}M$ structure

- a rare earth ternary complex of $[(CCl_3\text{-}C_6Cl_4SO_2)_2N]_{n2}M$ and triphenylphosphine
- a rare earth ternary complex of $[(CCl_3\text{-}C_6Cl_4SO_2)_2N]_{n2}M$ and triphenylphosphine oxide
- a rare earth ternary complex of $[(CCl_3\text{-}C_6Cl_4SO_2)_2N]_{n2}M$ and diphenyl sulfide
- a rare earth ternary complex of $[(CCl_3\text{-}C_6Cl_4SO_2)_2N]_{n2}M$ and diphenyl sulfoxide
- a rare earth ternary complex of $[(CCl_3\text{-}C_6Cl_4SO_2)_2N]_{n2}M$ and diphenylamine
- a rare earth ternary complex of $[(CCl_3\text{-}C_6Cl_4SO_2)_2N]_{n2}M$ and triphenylamine
- a rare earth ternary complex of $[(CCl_3\text{-}C_6Cl_4SO_2)_2N]_{n2}M$ and aniline
- a rare earth ternary complex of $[(CCl_3\text{-}C_6Cl_4SO_2)_2N]_{n2}M$ and pyridine
- a rare earth ternary complex of $[(CCl_3\text{-}C_6Cl_4SO_2)_2N]_{n2}M$ and bipyridine
- a rare earth ternary complex of $[(CCl_3\text{-}C_6Cl_4SO_2)_2N]_{n2}M$ and phenanthroline

* The complexes listed below, having a $[(C_xF_{2x+1}SO_2)\ (C_yF_{2y+1}SO_2)N]_{n2}M$ structure (wherein x and y are different form each other, and are each an integer from 1 to 10).

- a rare earth ternary complex of $[(C_xF_{2x+1}SO_2)\ (C_yF_{2y+1}SO_2)N]_{n2}M$ (x, y = 1 to 10) and triphenylphosphine
- a rare earth ternary complex of $[(C_xF_{2x+1}SO_2)\ (C_yF_{2y+1}SO_2)N]_{n2}M$ (x, y = 1 to 10) and triphenylphosphine oxide
- a rare earth ternary complex of $[(C_xF_{2x+1}SO_2)\ (C_yF_{2y+1}SO_2)N]_{n2}N$ (x, y = 1 to 10) and diphenyl sulfide
- a rare earth ternary complex of $[(C_xF_{2x+1}SO_2)\ (C_yF_{2y+1}SO_2)N]_{n2}M$ (x, y = 1 to 10) and diphenyl sulfoxide
- a rare earth ternary complex of $[(C_xF_{2x+1}SO_2)\ (C_yF_{2y+1}SO_2)N]_{n2}M$ (x, y = 1 to 10) and diphenylamine
- a rare earth ternary complex of $[(C_xF_{2x+1}SO_2)\ (C_yF_{2y+1}SO_2)N]_{n2}N$ (x, y = 1 to 10) and triphenylamine
- a rare earth ternary complex of $[(C_xF_{2x+1}SO_2)\ (C_yF_{2y+1}SO_2)N]_{n2}M$ (x, y = 1 to 10) and aniline
- a rare earth ternary complex of $[(C_xF_{2x+1}SO_2)\ (C_yF_{2y+1}SO_2)N]_{n2}M$ (x, y = 1 to 10) and pyridine
- a rare earth ternary complex of $[(C_xF_{2x+1}SO_2)\ (C_yF_{2y+1}SO_2)N]_{n2}M$ (x, y = 1 to 10) and bipyridine
- a rare earth ternary complex of $[(C_xF_{2x+1}SO_2)\ (C_yF_{2y+1}SO_2)N]_{n2}M$ (x, y = 1 to 10) and phenanthroline

* The complexes listed below, having a $[(C_6F_5SO_2)\ (C_xF_{2x+1}SO_2)N]_{n2}M$ structure (wherein x is an integer from 1 to 10)

- a rare earth ternary complex of $[(C_6F_5SO_2)\ (C_xF_{2x+1}SO_2)N]_{n2}M$ (x = 1 to 10) and triphenylphosphine
- a rare earth ternary complex of $[(C_6F_5SO_2)\ (C_xF_{2x+1}SO_2)N]_{n2}M$ (x = 1 to 10) and triphenylphosphine oxide
- a rare earth ternary complex of $[(C_6F_5SO_2)\ (C_xF_{2x+1}SO_2)N]_{n2}M$ (x = 1 to 10) and diphenyl sulfide
- a rare earth ternary complex of $[(C_6F_5SO_2)\ (C_xF_{2x+1}SO_2)N]_{n2}M$ (x = 1 to 10) and diphenyl sulfoxide
- a rare earth ternary complex of $[(C_6F_5SO_2)\ (C_xF_{2x+1}SO_2)N]_{n2}M$ (x = 1 to 10) and diphenylamine
- a rare earth ternary complex of $[(C_6F_5SO_2)\ (C_xF_{2x+1}SO_2)N]_{n2}M$ (x = 1 to 10) and triphenylamine
- a rare earth ternary complex of $[(C_6F_5SO_2)\ (C_xF_{2x+1}SO_2)N]_{n2}N$ (x = 1 to 10) and aniline
- a rare earth ternary complex of $[(C_6F_5SO_2)\ (C_xF_{2x+1}SO_2)N]_{n2}M$ (x = 1 to 10) and pyridine
- a rare earth ternary complex of $[(C_6F_5SO_2)\ (C_xF_{2x+1}SO_2)N]_{n2}N$ (x = 1 to 10) and bipyridine
- a rare earth ternary complex of $[(C_6F_5SO_2)\ (C_xF_{2x+1}SO_2)N]_{n2}M$ (x = 1 to 10) and phenanthroline

Examples of particularly preferable complexes include those listed below, in which x in the following formulas is an integer from 1 to 4.

- a rare earth ternary complex of $[(C_xF_{2x+1}SO_2)_2N]_{n2}M$ and triphenylphosphine,
- a rare earth ternary complex of $[(C_xF_{2x+1}SO_2)_2N]_{n2}M$ and triphenylphosphine oxide,
- a rare earth ternary complex of $[(C_xF_{2x+1}SO_2)_2N]_{n2}M$ and diphenyl sulfoxide,
- a rare earth ternary complex of $[(C_xF_{2x+1}SO_2)_2N]_{n2}M$ and diphenylamine,
- a rare earth ternary complex of $[(C_xF_{2x+1}SO_2)_2N]_{n2}M$ and triphenylamine,
- a rare earth ternary complex of $[(C_xF_{2x+1}SO_2)_2N]_{n2}M$ and pyridine,
- a rare earth ternary complex of $[(C_xF_{2x+1}SO_2)_2N]_{n2}M$ and bipyridine,

- a rare earth ternary complex of $[(C_xF_{2x+1}SO_2)_2N]_{n2}M$ and phenanthroline.

A complex represented by the following formula (4) or (5) is particularly preferable as the rare earth ternary complex represented by formula (1).

Formula (4):

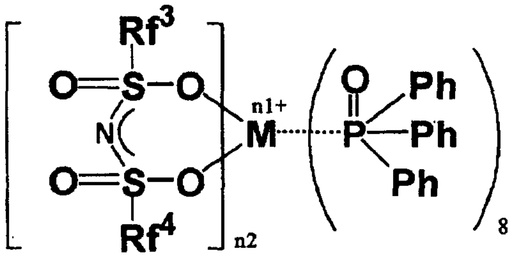

(4)

wherein M, n1, and n2 are as defined above; $Rf^3$ and $Rf^4$ are the same or different and are each a perfluoroalkyl group having 1 to 4 carbon atoms; and Ph is a phenyl group.

The complex represented by formula (4) is a rare earth ternary complex in which the ligand Z is represented by formula (A), and in formula (A), X is a phosphorus atom, and $R^1$, $R^2$, and $R^3$ are phenyl groups.

Formula (5):

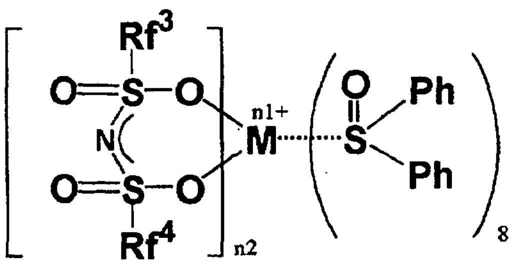

(5)

wherein M, n1, and n2 are as defined above; $Rf^3$ and $Rf^4$ are the same or different and are each a perfluoroalkyl group having 1 to 4 carbon atoms; and Ph is a phenyl group.

The complex represented by formula (5) is a rare earth ternary complex in which the ligand Z is represented by formula (A), and in formula (A), X is a sulfur atom, $R^1$ and $R^2$ are phenyl groups, and m3 is 0.

Compared to heretofore rare earth ternary complexes in which triphenylphosphine was coordinated to a β-diketone rare earth complex, the complexes represented by formulas (4) and (5) exhibit higher relative luminescence intensity, far beyond expectation, and their miscibility with various media is also greatly improved.

Process for producing the rare earth ternary complex

The rare earth ternary complex of the present invention can be prepared by the following two methods, for example.

1) Process for producing a ternary complex by reacting a rare earth binary complex represented by the following formula (2) with a compound Z'

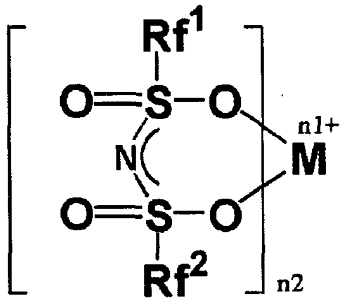

(2)

2) Process for producing a ternary complex by reacting the following three components

a) A compound represented by formula (6):

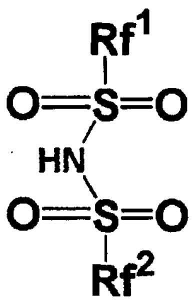

(6)

wherein $Rf^1$ and $Rf^2$ are as defined in formula (1) ,

b) the compound Z', and

c) a rare earth metal compound.

1) Process for producing a ternary complex by reacting a rare earth complex represented by the following formula (2) with a compound Z' in a solvent

This is a method for producing a rare earth ternary complex by mixing a compound represented by formula (2) with the compound Z'.

A compound represented by formula (2) can be obtained by a known method. One example is a method in which a rare earth metal compound is made to react with a compound represented by formula (6). Examples of the rare earth metal compound include the rare earth metal compounds that can be used in the "process for producing a ternary complex by reaction three components" described below.

Specific examples of the rare earth complex represented by formula (2) include the following complexes.

1) N-symmetrical complexes: rare earth complexes having a ligand in which two identical substituents are bonded to a nitrogen atom. In the following formulas, $C_xF_{2x-1}$ is a perfluoroalkenyl group, and $C_xCl_{2x-1}$ is a perchloro-alkenyl group.

- $[(C_xF_{2x+1}SO_2)_2N]_{n2}M$ (x = 1 to 22)
- $[(C_xCl_{2x+1}SO_2)_2N]_{n2}M$ (x = 1 to 22)
- $[(C_xF_{2x-1}SO_2)_2N]_{n2}M$ (x = 2 to 22)

- $[(C_xCl_{2x-1}SO_2)_2N]_{n2}M$ (x = 2 to 22)
- $[(C_6F_5SO_2)_2N]_{n2}M$
- $[(CF_3\text{-}C_6F_4SO_2)_2N]_{n2}M$
- $[(CCl_3\text{-}C_6Cl_4SO_2)_2N]_{n2}M$

2) N-assymmetrical complexes: rare earth complexes having a ligand in which two different substituents are bonded to a nitrogen atom. In the following formulas, $C_xF_{2x-1}$ and $C_yF_{2y-1}$ are perfluoroalkenyl groups, and $C_xCl_{2x-1}$ and $C_yCl_{2y-1}$ are perchloroalkenyl groups.

- $[(C_xF_{2x+1}SO_2)\ (C_yF_{2y+1}SO_2)N]_{n2}M$ (x and y are different from each other, and x, y = 1 to 22)
- $[(C_xCl_{2x+1}SO_2)\ (C_yCl_{2y+1}SO_2)N]_{n2}M$ (x and y are different from each other, and x, y = 1 to 22)
- $[(C_xF_{2x+1}SO_2)\ (C_yCl_{2y+1}SO_2)N]_{n2}M$ (x, y 1 to 22)
- $[(C_xF_{2x-1}SO_2)\ (C_yF_{2y-1}SO_2)N]_{n2}M$ (x and y are different from each other, and x, y = 2 to 22)
- $[(C_xCl_{2x-1}SO_2)\ (C_yCl_{2y-1}SO_2)N]_{n2}M$ (x and y are different from each other, and x, y = 2 to 22)
- $[(C_xF_{2x-1}SO_2)\ (C_yCl_{2y-1}SO_2)N]_{n2}M$ (x, y = 2 to 22)
- $[(C_6F_5SO_2)\ (C_xF_{2x+1}SO_2)N]_{n2}M$ (x = 1 to 22)
- $[(CF_3\text{-}C_6F_4SO_2)(C_xF_{2x+1}SO_2)N]_{n2}M$ (x = 1 to 22)
- $[(CCl_3\text{-}C_6Cl_4SO_2)\ (C_xF_{2x+1}SO_2)N]_{n2}M$ (x = 1 to 22)
- $[(C_6Cl_5SO_2)\ (C_xF_{2x+1}SO_2)N]_{n2}M$ (x = 1 to 22)
- $[(C_6F_5SO_2)\ (C_xCl_{2x+1}SO_2)N]_{n2}M$ (x = 1 to 22)
- $[(C_6Cl_5SO_2)\ (C_xCl_{2x+1}SO_2)N]_{n2}M$ (x = 1 to 22)

The following complexes are preferable as the rare earth complex represented by formula (2).

N-symmetrical complexes:

- $[(C_xF_{2x+1}SO_2)_2N]_{n2}M$ (x = 1 to 10)
- $[(C_xCl_{2x+1}SO_2)_2N]_{n2}M$ (x = 1 to 10)
- $[(C_6F_5SO_2)_2N]_{n2}M$
- $[(CCl_3\text{-}C_6Cl_4SO_2)_2N]_{n2}M$

N-assymmetrical complexes:

- $[(C_xF_{2x+1}SO_2)\ (C_yF_{2y+1}SO_2)N]_{n2}M$ (x and y are different from each other, and x, y = 1 to 10)
- $[(C_6F_5SO_2)\ (C_xF_{2x+1}SO_2)N]_{n2}M$ (x = 1 to 10)

The compound Z' used in the preparing of the complex is at least one compound selected from the group consisting of the following (A) to (D).

(A) a compound represented by the following formula (3) :

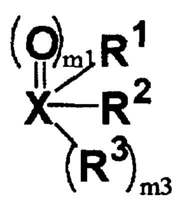

(3)

wherein $R^1$, $R^2$, and $R^3$ are the same or different and are each a hydrogen atom, a deuterium atom, an alkyl group having 1 to 20 carbon atoms, an alkyloxy group having 1 to 20 carbon atoms, an aromatic group, or an aryloxy group, and these groups may be substituted with deuterium; at least one of $R^1$, $R^2$, and $R^3$ is an aromatic group or an aryloxy group; X is a phosphorus or sulfur atom; m1 is 0 or 1; and when X is a phosphorus atom, m3

is 1, and when X is a sulfur atom, m3 is 0.

(B) a compound represented by the following formula (B'):

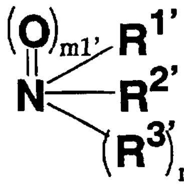

(B')

wherein $R^{1'}$, $R^{2'}$, and $R^{3'}$ are the same or different and are each a hydrogen atom, a deuterium atom, an alkyl group having 1 to 20 carbon atoms, an alkyloxy group having 1 to 20 carbon atoms, or an aromatic group, and these groups may be substituted with deuterium; at least one of $R^{1'}$, $R^{2'}$, and $R^{3'}$ is an aromatic group; m1' is 0 or 1; and m3' is 1.

(C) a nitrogen-containing aromatic compound with monodentate coordination to M

(D) a nitrogen-containing aromatic compound with bidentate coordination to M

In the formulas (3) and (B'), the definitions of X, $R^1$, $R^2$, $R^3$, $R^{1'}$, $R^{2'}$, $R^{3'}$, m1, m3, m1', and m3' are the same as defined in formulas (A) and (B).

Examples of nitrogen-containing aromatic compound with monodentate coordination to M include pyridine, pyrazine, pyrimidine, pyridazine, triazine, and the like. Pyridine is preferable.

Examples of the nitrogen-containing aromatic compound with bidentate coordination to M include bipyridine (such as 2,2'-bipyridine), phenanthroline (such as 1,10-phenanthroline), and the like. 2,2'-bipyridine and 1,10-phenanthroline are preferable.

There are no particular restrictions on the ratio in which the rare earth complex represented by formula (2) is mixed with the compound Z'. The amount of compound Z' per mole of the compound represented by formula (2) is usually about 1 to about 30 mol, and preferably about 2 to about 10 mol.

In this method, a solvent may be added as needed. Namely, the compound represented by formula (2) may be made to react with the compound Z' in a solvent.

The solvent is not limited, and any solvent can be used. Examples include protic solvents, aprotic solvents, and the like. Examples of protic solvents include water, lower alcohols (such as methanol, ethanol, n-propanol, isopropanol, n-butanol, isobutanol, tert-butanol, etc.), and the like. Examples of aprotic solvents include ketones (such as acetone, methyl ethyl ketone, etc.), ethers (such as diethyl ether, tetrahydrofuran, etc.), hydrocarbon solvents (such as n-hexane, cyclohexane, etc), chlorine-containing solvents (such as chloroform, methylene chloride, etc.), DMF (dimethylformamide), DMSO (dimethyl sulfoxide), and the like. Lower alcohols, ketones, DMF, DMSO, and the like are preferable.

The amount of solvent used is not limited, but is usually about 1 to about 100 weight parts, and preferably about 1 to about 20 weight parts, when the total amount of the rare earth ternary complex represented by formula (2) and the compound Z' is 1 weight part.

The system may be stirred as needed during the reaction. The reaction temperature is usually between room temperature and about 150°C, and preferably about 30 to about 100°C. The reaction time is usually about 0.1 to about 30 hours, and preferably about 0.1 to about 6 hours.

After the reaction, the solution can be concentrated as needed, and the obtained residue can be treated as needed by a known method such as liquid-liquid extraction, precipitation, or the like method to obtain a rare earth ternary complex. The obtained rare earth ternary complex may be further refined as needed by a known refining method such as recrystallization, column chromatography, sublimation, or the like.

2) Process for producing a ternary complex by reacting three components

This is a method for producing a rare earth ternary complex by mixing a compound represented by formula (6), the compound Z', and a rare earth metal compound.

The compound represented by formula (6) is a precursor of the rare earth complex represented by formula (2), and is a compound in which the rare earth atoms of the rare earth complex represented by formula (2) have been substituted with hydrogen atoms. The compound represented by formula (6) can be purchased commercially, or it can be prepared by a known method, such as the method described in WO98/40388 or the like.

Examples of the rare earth metal compound used in the producing here include rare earth metal oxides, rare

earth metal hydroxides, rare earth metal alkoxides, rare earth metal amides, rare earth metal salts and the like. The rare earth metal compound may be used either alone, or in a combination of two or more.

Examples of a rare earth metal oxide include $M_2O_3$ (M is a rare earth atom; the same applies hereinafter) including a trivalent rare earth metal, but MO, $M_4O_7$, and other such oxides can also be used.

Examples of a rare earth metal hydroxide include $M(OH)n_a$, wherein n is an integer from 2 to 4, and the like.

Examples of a rare earth metal alkoxide include $M(OR^1)n_b$, wherein $R^1$ is an alkyl group having 1 to 8 carbon atoms, and $n_b$ is an integer from 2 to 4, and the like.

Examples of a rare earth metal amide include M $(NR^aR^b)_3$, wherein $R^a$ and $R^b$ are the same or different, and are each a hydrogen, an alkyl group having 1 to 10 carbon atoms, a phenyl group, or the like.

Examples of a rare earth metal salt include $M^{L+}(Za)n_c$, wherein Za is a chloride ion, bromide ion, iodide ion, fluoride ion, 1/2 sulfuric acid ion, nitric acid ion, monocarboxylic acid ion (such as an acetic acid ion, and the like), 1/2 dicarboxylic acid ion (such as a 1/2 oxalic acid ion, 1/2 succinic acid ion, 1/2 malonic acid ion, and the like), 1/3 tricarboxylic acid ion (such as a 1/3 citric acid ion, and the like), 1/3 phosphoric acid ion, $n_c$ is an integer from 2 to 4, and L is an integer from 2 to 4, and the like salt. In a rare earth metal salt, L is usually an integer from 2 to 4, and preferably 3.

The amount of the rare earth metal salt used is usually about 1 to about 10 mol, and preferably about 1.05 to about 3 mol, per mole of the compound represented by formula (6).

The amount of the compound Z' used is usually about 1 to about 30 mol, and preferably about 2 to about 10.mol, per mole of the compound represented by formula (6).

In this method, a solvent may be added as needed. Namely, the compound represented by formula (6), the compound Z', and a rare earth metal compound may be reacted in a solvent.

The solvent used to prepare the rare earth ternary complex is not limited, and any solvent can be used. Examples include protic solvents, aprotic solvents, and the like. Examples of protic solvents include water; lower alcohols such as methanol, ethanol, and the like. Examples of aprotic solvents include ketones such as acetone, methyl ethyl ketone, and the like; ethers such as diethyl ether, tetrahydrofuran, and the like; halogen-containing solvents such as chloroform, methylene chloride, and the like; DMSO; DMF; and the like. Of these, it is preferable to use a solvent capable of simultaneously dissolving all three components comprising the compound represented by formula (6), the rare earth metal compound, and the compound Z'. Examples of such solvents include the mixed solvents such as the solvent including water and lower alcohol, water and acetone, water and DMF, and water and DMSO, and the like mixed solvent.

The amount of the solvent used is usually about 1 to about 100 weight parts, and preferably about 1 to about 20 weight parts, when the total amount of the compound represented by formula (6), the rare earth metal compound, and the compound Z' is 1 weight part.

The system may be stirred as needed during the reaction. The reaction temperature is usually between room temperature and about 150°C, and preferably about 30 to about 100°C. The reaction time is usually about 0.1 to about 100 hours, and preferably about 0.1 to about 20 hours.

After the stirring, the solution can be concentrated as needed, and the obtained residue can be treated as needed by a known method such as liquid-liquid extraction, precipitation, or the like method to obtain a rare earth ternary complex. The obtained rare earth ternary complex may be further refined as needed by a known refining method such as recrystallization, column chromatography, sublimation, or the like.

The rare earth ternary complex represented by formula (1) can be prepared by the above-mentioned two methods, for example.

Optically functional material

The rare earth ternary complex of the present invention can be used as an optically functional material.

Examples of how the rare earth ternary complex of the present invention is made to emit light include a method in which the rare earth ternary complex of the present invention is dissolved, dispersed, or suspended in a medium (such as various solvents, a polymer matrix, and the like), and is made to emit light by irradiation with light of a specific wavelength, or the like method.

The medium containing no hydrogen atoms is preferable.

Examples of solvents used as the medium include ketones such as acetone, methyl ethyl ketone, and the like; ethers such as diethyl ether, tetrahydrofuran, isopropyl ether, dioxane, and the like; aromatic hydrocarbons such as benzene, toluene, and the like; halogenated hydrocarbons such as methylene chloride, chloroform, carbon tetrachloride, and the like; amides such as acetamide, formamide, DMF, diethylformamide, and the like; DMSO; esters such as ethyl acid, methyl acetate, and the like; glycols such as ethylene glycol, propylene glycol, and the like, and the like solvent. It is preferable for the solvent used as the medium to be an organic solvent substituted with deuterium, such as deuterated methanol, deuterated acetone, deuterated tetrahydrofuran, DMF-$d_6$, and the like.

The concentration in which the rare earth ternary complex of the present invention dissolved in the solvent is

usually about 0.0001 to about 1 mol/L, and preferably about 0.05 to about 0.5 mol/L, and more preferably about 0.01 to about 0.3 mol/L.

It is preferable for the polymer matrix used as the medium to be a semi-transparent or transparent composition when blended with the rare earth ternary complex. Examples include polymethacrylate (PMA), polymethyl methacrylate (PMMA); poly(hexafluoroisopropyl methacrylate) (P-iFPMA), poly(hexafluoro-n-propyl methacrylate) (P-nFPMA), and other such fluorine-containing polymethacrylates; polyacrylates; fluorine-containing polyacrylates typified by polyfluoroisopropyl acrylate; polystyrene, polyethylene, polypropylene, polybutene, and other such polyolefins; fluorine-containing polyolefins; polyvinyl alcohols; polyvinyl ethers; fluorine-containing polyvinyl ethers typified by poly(perfluoropropoxy)vinyl ether; polyvinyl acetate, polyvinyl chloride; copolymers comprising two or more of the monomers that make up the above-mentioned polymers; cellulose; polyacetal; polyester; polycarbonate; epoxy resins; polyamide resins; polyimide resins; polyurethane; perhalogenated ion exchange resins (such as perfluorinated ion exchange resins (Nafion, etc.), and the like); petroleum resins; rosins; silicon resins, and so on. Preferable polymer matrix includes, for example, polymethyl methacrylate; fluorine-containing polymethacrylate; polyacrylate; fluorine-containing polyacrylate; polyolefins such as polystyrene, polyethylene, polypropylene, polybutene, and the like; polyvinyl ether; fluorine-containing polyvinyl ether; copolymers comprising two or more of the monomers that make up the above-mentioned polymers; epoxy resins; perhalogenated ion exchange resins (such as perfluorinated ion exchange resins (Nafion, etc.), and the like).

As long as the function of the polymer matrix is not compromised, additives may be added to the polymer matrix for the purpose of improving the characteristics thereof. Specific examples of such additives include dibutyl phthalate, dioctyl phthalate, and other such phthalic diesters; dioctyl adipate and other such dibasic acid diesters; pentaerythritol tetrabenzoate and other such polyol esters; dispersants containing a surfactant such as rosin acid soap, stearic acid soap, oleic acid soap, sodium lauryl sulfate, sodium diethylhexylsulfosuccinate, and sodium dioctylsulfosuccinate; alkyl sulfonate, alkyl ether carboxylic acids, and other such anionic antistatic agents; polyethylene glycol derivatives, sorbitan derivatives, and other such nonionic antistatic agents; quaternary ammonium salts, alkylpyridiums, and other such cationic antistatic agents; talc, metal salts of fatty acids, sorbitan-based crystallization nucleators; butylhydroxyphenol and other such phenol-based antioxidants; thioether-based antioxidants; phosphorus-based antioxidants; pigments; photostabilizers; crosslinking agents; crosslinking promoter; flame retardants; processing aids, and the like. The amount of the additives used can be suitably determined according to the type of additive, but is usually about 0.01 to about 10 weight parts per 100 weight parts of the polymer matrix.

There are no particular restrictions on the method for dispersing or suspending the rare earth ternary complex of the present invention in the polymer matrix. Examples include (1) a method in which a rare earth complex is mixed into a molten polymer matrix, (2) a method in which a rare earth complex is dispersed in a polymer micropowder, then melted, (3) a method in which a monomer serving as the raw material for the polymer matrix, a ternary complex, and a polymerization initiator such as azobisisobutyronitrile (AIBN), lauroyl peroxide or the like are reacted as a mixture, (4) a cast method in which a rare earth complex is mixed into a polymer solution that is useful for producing a polymer film, and the solvent is then removed, (5) spin coating method, (6) vapor codeposition method and the like.

When the rare earth ternary complex of the present invention is dispersed or suspended in a polymer matrix, the amount of the rare earth ternary complex used is usually about 0.001 to about 20 weight parts, and preferably about 0.1 to about 10 weight parts, per 100 weight parts of polymer matrix.

When the rare earth ternary complex of the present invention is made to emit light, the wavelength of the excitation light can be suitably set according to the kind of rare earth metal: M contained in the complex. For instance, when neodymium is used as the rare earth atom, light with a wavelength of about 1060 nm is emitted upon irradiation with light having a wavelength of about 585 nm as the excitation light. Similarly, when europium or terbium is used as the rare earth atom, light with a wavelength of about 618 nm and about 545 nm is emitted upon irradiation with light having a wavelength of about 394 nm and about 325 nm as the excitation light, respectively.

The excitation wavelength may be set by measuring the wavelength of absorption maximum of the ternary complex in the medium. The wavelength in the UV-visible range is preferable as the wavelength of absorption maximum, and about 180 to about 500 nm is particularly preferable. For example, the excitation wavelength may be set to usually within about 50 nm, and preferably within about 20 nm, above or below the wavelength of absorption maximum.

The effects of the rare earth ternary complex of the present invention are summarized below.

1) To prepare of a ternary complex, the number of moles of bound water per mole of complex is reduced.

Heretofore sulfonimide-based rare earth complexes were deliquescent, and when they were used as a luminescent material, water was removed very carefully. On the other hand, the rare earth ternary complex of the present invention is easier to handle, because bond water is difficult to bind to the present complex.

2) Miscibility in various media is improved.

To prepare of a ternary complex, miscibility in media with low polarity is improved, which means that there is a much broader range of application in use as a luminescent material.

3) Relative luminescence intensity is remarkably increased.

The complex of the present invention exhibits extremely high luminescence intensity. Compared to the rate of increase in the luminescence intensity when the ligand Z is introduced into a known β-diketone type of complex, the rate of increase in the luminescence intensity is far greater when the ligand Z is introduced into the complex represented by formula (2).

4) Relative luminescence intensity is increased even further in a polymer matrix.

The effect in 3) above is even more pronounced when light is emitted in a polymer matrix. For example, a europium ternary complex having triphenylphosphine oxide (TPPO) as the ligand Z exhibits a luminescence intensity up to or more than 10,000 times that of a complex having no TPPO as a ligand. Thus, the amount of rare earth ternary complex used can be greatly reduced when the complex is used as a luminescent material or other optically functional material.

It is not fully understood why there is such a marked increase in the relative luminescence intensity of the rare earth ternary complex of the present invention, but it is supposed that the two sulfur atoms in the rare earth ternary complex play a part.

With a complex in which the ligand Z is introduced into a known binary complex having no sulfur atoms, such as a complex obtained by introducing triphenylphosphine oxide into $Eu(HFA)_3$, the increase in luminescence intensity is only a few times that prior to the introduction of the triphenylphosphine oxide.

The rare earth ternary complex of the present invention can be used along with the ligand Z. Because the ligand Z interacts with the ternary complex of the present invention represented by formula (1), there is an even greater increase in luminescence intensity. The amount of the ligand Z used is usually about 0.01 to about 100 mol, and preferably about 0.1 to about 10 mol, per mole of complex represented by formula (1).

Mechanoluminescence

The rare earth ternary complex of the present invention can be used as a mechanoluminescence material. The term mechanoluminescence as used here refers to a phenomenon whereby physical force (such as pressure, vibration energy or the like) imparted to the rare earth ternary complex is converted into optical energy, causing the complex to emit light.

Examples of how mechanoluminescence is occurred include a method in which physical pressure is applied to the complex, when the rare earth ternary complex is in the form of a powder or thin film, the complex has been dispersed or suspended in a polymer thin film, or the like.

Examples of how a rare earth ternary complex is made into a thin film, that is, how a thin film comprising a rare earth ternary complex is obtained, include a method in which a rare earth ternary complex is dissolved in acetone or other organic solvents, and the solution is made into a film and dried, and the like method. The thickness of the film is not limited, but it is usually about 1 to about 20 μm, and preferably about 1 to about 10 μm. The methods for manufacturing a thin film comprising the above-mentioned rare earth ternary complex is not limited, but Examples of the methods include casting, spin coating, roller transfer, and the like.

The following is an example of the method for obtaining a film dispersed or suspended the ternary complex in a polymer. A polymer solution in which a rare earth ternary complex is uniformly dispersed is prepared by adding the rare earth ternary complex to a mixture of a solvent and a polymer, and stirring as needed, or the like method. This polymer solution is then made into a film and dried to manufacture a polymer film in which a rare earth ternary complex is uniformly dispersed or suspended.

The polymer is not limited, and various types can be used. Examples of polymers include polycarbonates, polyether imides, polyether ether ketones, polysulfones, polymethylpentene, polymethyl methacrylate, polyolefins (such as polystyrene, polyethylene, polypropylene, polybutene, etc.), liquid crystal polymers, and the like.

The following are further examples of polymers: polymethacrylate (PMA); poly(hexafluoroisopropyl methacrylate) (P-iFPMA), poly(hexafluoro-n-propyl methacrylate) (P-nFPMA), and other such fluorine-containing polymethacrylates; polyacrylates; fluorine-containing polyacrylates typified by polyfluoroisopropyl acrylate; fluorine-containing polyolefins; polyvinyl alcohols; polyvinyl ethers; fluorine-containing polyvinyl ethers typified by poly(perfluoropropoxy)vinyl ether; polyvinyl acetate, polyvinyl chloride; copolymers comprising of two or more of the monomers that make up the above-mentioned polymers; cellulose, polyacetals; polyesters; epoxy resins; polyamide resins; polyimide resins; polyurethanes; perhalogenated ion exchange resins (such as perfluorinated ion exchange resins (Nafion, etc.), and the like); petroleum resins; rosin; silicon resins, and the like.

The amount of the rare earth ternary complex dispersed or suspended in the polymer is about 5 to about 20 weight parts, and preferably about 10 to about 20 weight parts, per 100 weight parts of polymer (or 100 weight parts of monomer corresponding to 100 weight parts of polymer). The thickness of the polymer film is not limited, but it is usually about 1 to about 20 μm, and preferably about 1 to about 10 μm. The polymer concentration in the polymer

solution used to manufacture the polymer film is about 5 to about 30 wt%, and preferably about 10 to about 15 wt%.

Examples of solvents include butyl acetate, tetrahydrofuran (THF), toluene, acetonitrile, methyl ethyl ketone, xylene, and the like.

Examples of the method for preparing a film by using a polymer solution containing the rare earth ternary complex include casting, spin coating, roller transfer, and the like.

Mechanoluminescence is a phenomenon whereby physical force (such as vibration, shock, or the like) is directly converted into optical information, so the complex of the present invention can be utilized as a pressure/light conversion element, a pressure sensor, or the like.

Composition

The composition of the present invention includes the rare earth binary complex represented by formula (2) and the compound Z'. The compound Z' is at least one compound selected from the group consisting of the above-mentioned (A) to (D). In this composition, the rare earth binary complex represented by formula (2) and the compound Z' may form the rare earth ternary complex represented by formula (1).

The composition of the present invention can be used as an optically functional material.

The ratio between the rare earth binary complex represented by formula (2) and the compound Z' in the composition is not limited. The amount of the compound Z' contained is usually about 10 to about 5000 weight parts, and preferably about 100 to about 3000 weight parts, per 100 weight parts of the rare earth birnary complex.

A solvent may be contained in the composition. The amount of the solvent contained is not limited. The solvent content is usually about 100 to about 10,000 weight parts, and preferably about 300 to about 3000 weight parts, per 100 weight parts of the rare earth binary complex represented by formula (2).

Examples of solvents contained in the composition include ketones such as acetone, methyl ethyl ketone, and the like; ethers such as diethyl ether, tetrahydrofuran, isopropyl ether, dioxane, and the like; aromatic hydrocarbons such as benzene, toluene, and the like; halogenated hydrocarbons such as methylene chloride, chloroform, carbon tetrachloride, and the like; amides such as acetamide, formamide, DMF, diethylformamide, and the like; DMSO; esters such as ethyl acid, methyl acetate, and the like; glycols such as ethylene glycol, propylene glycol and the like; and the like solvent. Other examples of solvents include organic solvents substituted with deuterium, such as deuterated methanol, deuterated acetone, deuterated tetrahydrofuran, DMF-$d_6$ and the like.

The composition may also. contain a polymer matrix or a monomer that serves as the raw material for a polymer matrix. The amount of the polymer matrix or monomer contained is not limited, but it is usually about 10 to about 10,000 weight parts, and preferably about 100 to about 3000 weight parts, per 100 weight parts of the rare earth binary complex represented by formula (2).

Examples of the polymer matrix contained in the composition include polymethacrylate (PMA), poly(methyl methacrylate) (PMMA); poly(hexafluoroisopropyl methacrylate) (P-iFPMA), poly(hexafluoro-n-propyl methacrylate) (P-nFPMA), and other such fluorine-containing polymethacrylates; polyacrylates; fluorine-containing polyacrylates typified by polyfluoroisopropyl acrylate; polystyrene, polyethylene, polypropylene, polybutene, and other such polyolefins; fluorine-containing polyolefins; polyvinyl alcohols; polyvinyl ethers; fluorine-containing polyvinyl ethers typified by poly (perfluoropropoxy)vinyl ether; polyvinyl acetate, polyvinyl chloride; copolymers comprising two or more of the monomers that make up the above-mentioned polymers; cellulose; polyacetal; polyesters; polycarbonates; epoxy resins; polyamide resins; polyimide resins; polyurethanes; perhalogenated ion exchange resins (such as perfluorinated ion exchange resins (Nafion, etc.), and the like), petroleum resins; rosin; silicon resins, and the like polymer matrix. Examples of preferable polymer matrixes include poly(methyl methacrylate); fluorine-containing polymethacrylates; polyacrylates; fluorine-containing polyacrylates; polystyrene, polyethylene, polypropylene, polybutene, and other such polyolefins; polyvinyl ethers; fluorine-containing polyvinyl ethers; copolymers comprising two or more of the monomers that make up the above-mentioned polymers; epoxy resins; perhalogenated ion exchange resins (such as perfluorinated ion exchange resins (Nafion, etc.), and the like).

The above-mentioned additives may be added to the composition of the present invention for the purpose of improving the characteristics of the polymer matrix. The amount of the additives added can be suitably determined according to the kind of additives, but is usually about 0.01 to about 10 weight parts per 100 weight parts of polymer matrix.

The composition of the present invention may also be molded. Any known molding method can be employed as the method for molding. Examples include a method in which molding is performed at the same time a rare earth complex-containing resin composition is prepared, a method in which a rare earth complex-containing resin composition is prepared first and then re-melted and molded, and the like. Molding processes that can be employed include injection molding, extrusion molding, blow molding, compressed air molding, rotary molding, film molding, and other such known molding methods.

The rare earth complex can also be added in a high concentration to a polymer matrix and then molded by

extrusion molding or the like to create a master batch.

The shape of the molded article is not limited, but examples include the form of a rod, film, sheet, cylinder, disk, oval, and the like. Alternatively, the article may have a special shape such as that of a toy, ornament or the like. Examples include a star shape, polygonal shape, and the like.

According to the present invention, a sulfonimide-based rare earth complex represented by formula (2) is made into a ternary complex, which yields a complex having remarkable greater luminescence intensity. The rare earth ternary complex of the present invention has high luminescence intensity and optical conversion efficiency, and can therefore be used to advantage as an optically functional material such as a luminescent material, mechanoluminescence material, or the like. The rare earth ternary complex of the present invention is useful in applications such as optical fibers, lenses, pressure sensors, lasers, and the like.

In particular, when a complex is dispersed in a polymer matrix, the luminescence intensity is remarkably higher than before the introduction of the ligand Z. Also, since producing a ternary complex improves miscibility in various media such as a polymer matrix, and the like, it affords broader application as an optically functional material.

## BEST MODE FOR CARRYING OUT THE INVENTION

The present invention is described by giving examples and comparative examples, but is not limited to or by the examples given below. The various properties were measured using the following equipment.

$^{19}$F-NMR (solvent: $CD_3OD$, reference reagent: hexafluorobenzene) and $^1$H-NMR (solvent: $CDCl_3$, reference reagent: tetramethylsilane) were measured using an NMR EX-270 from JEOL.

The IR spectrum was measured by KBr method using a 1720-X from Perkin-Elmer.

The temperature at which water molecules (bound water) were dissociated from the complex was measured using a DSC-50 from Shimadzu Corporation.

The number of moles of bound water per mole of complex was measured using a TG-DTA 2000 from MAC Science.

Elemental analysis was performed using a 240C from Perkin-Elmer.

UV absorption characteristics were measured with a UV-2100 from Shimadzu Corporation.

The intensity of luminescence and the luminescence quantum yield, the quotient of dividing the number of photons emitted from a sample by the number of photons absorbed into the sample, were measured with an SS-25 from JASCO Corporation.

The miscibility of the rare earth complex with various media was evaluated as follows.

1) Miscibility with solvents

The rare earth complex serving as the sample was added in a concentration of 5 wt% relative to various media, and the miscibility was visually evaluated according to the following criteria.

◎ : dissolves at room temperature
○: dissolves when heated for 5 minutes at 40°C
Δ : dissolves under heating, but cooling precipitates crystals
× : does not dissolve

2) Miscibility with a polymer matrix

The rare earth complex serving as the sample was added in the concentrations listed with the evaluation criteria below relative to various media, and the miscibility was visually evaluated according to the following criteria.

◎ : dissolves up to 30 wt%
○ : dissolves up to 5 wt%
Δ : disperses up to 5 wt%, but there is turbidity
× : repelled at the polymer surface

PMS stands for $[CF_3SO_2NSO_2CF_3]^-$, PES stands for $[C_2F_5SO_2NSO_2C_2F_5]^-$, and PBS stands for $[C_4F_9SO_2NSO_2C_4F_9]^-$.

Producing Example 1

Synthesis of $Eu(PMS)_3$

A 85.5 g quantity of commercially available $CF_3SO_2NHSO_2CF_3$ (made by Fluka) was dissolved in 30 mL of distilled water, 18.7 g of $Eu_2O_3$ was added, and the system was stirred for 3 days at room temperature. The water was distilled off, after which the precipitated solids were washed with methylene chloride, and the resulting solids were dissolved in methanol and filtered to remove the unreacted $Eu_2O_3$. The methanol was distilled off from the filtrate to give the targeted $Eu(PMS)_3$ in the form of a white solid (100 g, yield: 99%). The $^{19}$F-NMR, $^1$H-NMR, UV absorption characteristics, and IR spectrum of the obtained $Eu(PMS)_3$ were measured, the results are given below.

$^{19}$F-NMR: -77.51 ppm

$^1$H-NMR: none (2.06 ppm as bound water)

UV absorption characteristics: 394 nm ($^7F_0 \rightarrow {}^5L_6$), 465 nm ($^7F_0$ -> $^5D_2$)

IR: 1332 (S=O), 1205, 1142 (C=F), 1056 (S=O) cm$^{-1}$

Also, $Nd(PMS)_3$, $Yb(PMS)_3$, and $Tb(PMS)_3$ were synthesized in the same manner as in Producing Example 1, except that $Nd_2O_3$, $Yb_2O_s$, and $Tb_4O_7$, respectively, were used instead of the $Eu_2O_3$.

Producing Example 2

Synthesis of $C_4F_9SO_2NHSO_2C_4F_9$

A 30 mL quantity of THF, 3.3 g (28 mmol) of $CF_3CONH_2$ (made by Wako Pure Chemical Industries), and 10.0 g (72 mmol) of potassium carbonate (made by Kanto Kagaku) were put in a 100 mL three-necked flask equipped with a cooling pipe, and the contents were stirred for 1 hour at room temperature under a nitrogen atmosphere. A 8.5 g (28 mmol) quantity of $C_4F_9SO_2F$ (made by Tokyo Kasei Kogyo Co. Ltd.) was then added, and the system was refluxed for 3 hours. At this point, the reaction solution was analyzed to confirm that $C_4F_9SO_2NH_2$ had been produced. A 8.5 g (28 mmol) quantity of $C_4F_9SO_2F$ was then added to the reaction solution, and the system was refluxed for another 3 hours.

The THF was then distilled off under reduced pressure, after which the residue was dissolved in acetone, and insoluble salts such as $K_2CO_3$ and KF were filtered off. The acetone was then distilled off, and the resulting solids were washed with ether and recrystallized in ethanol to give 13.4 g of $C_4F_9SO_2NKSO_2C_4F_9$ in the form of colorless acicular crystals (yield: 77%). This salt was then stirred for 1 hour in a 25% $H_2SO_4$ aqueous solution, after which the reaction solution was extracted with ether. The ether layer was distilled off, and then the residue was sublimed at 100°C and 1.32 MPa to obtain the titled compound. The $^{19}$F-NMR and $^1$H-NMR were measured and the results for the obtained compound are given below.

$^{19}$F-NMR: -79.37 (t, 6F, $CF_3$), -111.57 (t, 4F, $CF_2$), - 119.29 (br, 4F, $CF_2$), -124.35 (br, 4F, $CF_2$) ppm

IR: 1347 (S=O), 1235 (C-F), 1201 (C-F), 1126 (S-O) cm$^{-1}$

$Eu(PBS)_3$ was synthesized in the same manner as in Producing Example 1, except that the $C_4F_9SO_2NHSO_2C_4F_9$ obtained by the above method was used instead of $CF_3SO_2NHSO_2CF_3$.

Example 1

Synthesis of $Eu(PMS)_3(TPPO)_8$

A 20 g quantity of $Eu(PMS)_3$ was added to 100 mL of isopropanol, then 43 g (16 times the molar amount of the $Eu(PMS)_3$) of triphenylphosphine oxide (hereinafter referred to as TPPO) was added, and the system was refluxed for 3 hours, and then gradually cooled. The resulting white powder was filtered off. The white powder thus obtained was washed with hot n-hexane, and recrystallized with water-methanol to obtain 44 g of $Eu(PMS)_3(TPPO)_8$ in the form of white acicular crystals (yield: 85%). The TG-DTA was measured for these crystals, and it was found from the weight reduction at 100°C that the number of moles of bound water per mole of complex was 2.93. The IR, NMR, UV absorption characteristics, and elemental analysis results for the obtained $Eu(PMS)_3(TPPO)_8(H_2O)_{2.93}$ are given below.

IR: 3061 (C-H st.), 1439 ($C_6H_5$ st.), 1355 (S=O st., P=O st., 1193 (C-F st.), 1122 (C-F st.), 1060 (S-O st.) cm$^{-1}$

$^1$H-NMR: 7.26. (br, $24C_6H_5$) ppm

UV absorption characteristics: 232 nm (K absorption band), 266 nm (B absorption band), 394 nm ($^7F_0 \rightarrow {}^5L_6$), 465 nm ($^7F_0 \rightarrow {}^5D_2$)

Elemental analysis: C150H124N3O22F18P8S6Eu, M.W. 3254, calculated values C: 55.31, H: 3.81, N: 1.29%, found values C: 55.97, H: 4.06, N: 1.16%.

$Eu(PMS)_3(TPPO)_8(H_2O)_{2.93}$ obtained by the above synthesis method was dried in vacuo for 3 hours at 140°C and a reduced pressure of 0.66 MPa. TG-DTA measurement for the resulting white powder was carried out to obtain

a weight reduction at 100°C. It was found from the obtained value that the number of moles of bound water per mole of complex was 0.18. The results of the elemental analysis of Eu $(PMS)_3(TPPO)_8(H_2O)_{0.18}$ are given below.

Elemental analysis: C150H120N3O20F18P8S6Eu, M.W. 3218, calculated values C: 55.93, H: 3.72, N: 1.31%, found values C: 57.35, H: 3.88, N: 1.19%.

Also, $Eu(PBS)_3(TPPO)_8$ was prepared in the same manner as in Example 1, except that the $Eu(PBS)_3$ was used instead of $Eu(PMS)_3$.

Example 2

Synthesis of $Nd(PMS)_3(TPPO)_8$

$Nd(PMS)_3(TPPO)_8$ was prepared in the same manner as in Example 1, except that 20 g of $Nd(PMS)_3$ was used instead of the $Eu(PMS)_3$ (yield after drying: 43 g (83%)).

The result of IR measurement and the number of moles of bound water per mole of complex for the obtained $Nd(PMS)_3(TPPO)_8$ are given below. The number of moles of bound water per mole of complex expresses the values for a sample dried in the same manner as in Example 1, and a sample prior to drying.

IR: 3060 (C-H st.), 1439 ($C_6H_5$ st.), 1354 (S=O st., P=O st., 1197 (C-F st.), 1143 (C-F st.), 1061 (S-O st.) $cm^{-1}$

Number of moles of bound water per mole of complex: 3.41 (before drying) and 0.24 (after drying)

Example 3

Synthesis of $Yb(PMS)_3(TPPO)_8$

$Yb(PMS)_3(TPPO)_8$ was prepared in the same manner as in Example 1, except that 20 g of $Yb(PMS)_3$ was used instead of $Eu(PMS)_3$. Yield after drying: 42 g (81%).

The result of IR measurement and the number of moles of bound water per mole of complex for the obtained $Yb(PMS)_3(TPPO)_8$ are given below. The number of moles of bound water per mole of complex expresses the values for a sample dried in the same manner as in Example 1, and a sample prior to drying.

IR: 3063 (C-H st.), 1440 ($C_6H_5$ st.), 1354 (S=O st., P=O st.), 1201 (C-F st.), 1143 (C-F st.) 1060 (S-O st.) $cm^{-1}$

Number of moles of bound water per mole of complex: 1.52 (before drying) and 0.27 (after drying)

Example 4

Synthesis of $Tb(PMS)_3(TPPO)_8$

$Tb(PMS)_3(TPPO)_8$ was prepared in the same manner as in Example 1, except that 20 g of $Tb(PMS)_3$ was used instead of $Eu(PMS)_3$. Yield after drying: 40 g (77%).

The result of IR measurement and the number of moles of bound water per mole of complex for the obtained $Tb(PMS)_3(TPPO)_8$ are given below. The number of moles of bound water per mole of complex expresses the values for a sample dried in the same manner as in Example 1, and a sample prior to drying.

IR: 3057 (C-H st.), 1439 ($C_6H_5$ st.), 1354 (S=O st., P=O st.), 1186 (C-F st.), 1142 (C-F st.), 1060 (S-O st.) $cm^{-1}$

Number of moles of bound water per mole of complex: 1.27 (before drying) and 0.08 (after drying)

Example 5

Synthesis of $Eu(PMS)_3(DPSO)_8$

A 10 g quantity of $Eu(PMS)_3$ was added to 100 mL of ethanol, 31.4 g (16 times the molar amount of the Eu $(PMS)_3$) of diphenyl sulfoxide (hereinafter referred to as DPSO) was added, and the system was refluxed for 3 hours. The system was then gradually cooled, and the resulting white powder was filtered off. The obtained white powder was recrystallized in ethanol to obtain 18 g of $Eu(PMS)_3(DPSO)_8$ in the form of white crystals (yield: 83%).

These crystals were subjected to DSC and TG-DTA measurement, which confirmed that water was not coordinated. The IR, NMR, UV absorption characteristics, and elemental analysis results for the obtained $Eu(PMS)_3$ $(DPSO)_8$ are given below.

IR: 3061 (C-H st.), 1439 ($C_6H_5$ st.), 1355 (S=O st.), 1193 (C-F st.), 1122 (C-F st.), 1060 (S-O st.) $cm^{-1}$

$^1$H-NMR: 7.25, 7.14 (br, $16C_6H_5$) ppm

UV absorption characteristics: 231 nm (K absorption band), 266 nm (B absorption band), 394 nm ($^7F_0$ -> $^5L_6$), 465 nm ($^7F_0 \rightarrow {}^5D_2$)

Elemental analysis: C102H80O20N3S14F18Eu, M.W. 2610, calculated values C: 46.90, H: 3.06, N: 1.61%, found values C: 47.08, H: 3.12, N: 1.67%.

Example 6

Synthesis of $Tb(PMS)_3(DPSO)_8$

$Tb(PMS)_3(DPSO)_8$ was obtained in the same manner as in Example 5, except that 10 g of $Tb(PMS)_3$ was used instead of the $Eu(PMS)_3$ (yield: 16 g (74%)). The result of IR measurement for the obtained $Tb(PMS)_3(DPSO)_8$ is given below.

IR: 3064 (C-H st.), 1447 ($C_6H_5$ st.), 1353 (S=O st.), 1201 (C-F st.), 1140 (C-F St.), 1056 (S-O St.) $cm^{-1}$

Example 7

Synthesis of $Nd(PMS)_3(DPSO)_8$

$Nd(PMS)_3(DPSO)_8$ was obtained in the same manner as in Example 5, except that 10 g of $Nd(PMS)_3$ was used instead of $Eu(PMS)_3$ (yield: 18 g (83%)). The result of IR measurement for the obtained $Nd(PMS)_3(DPSO)_8$ is given below.

IR: 3065 (C-H st.), 1448 ($C_6H_5$ st.), 1351 (S=O st.), 1201 (C-F st.), 1140 (C-F st.), 1054 (S-O st.) $cm^{-1}$

Example 8

Synthesis of $Eu(PMS)_3(Phen)_m$

A 19 g quantity of $Eu(PMS)_3$ was added to 100 mL of isopropanol, 16 g of 1,10-phenanthroline (hereinafter referred to as Phen) was added, and the system was refluxed for 3 hours. The system was then gradually cooled, and the resulting white powder was filtered off. The obtained white powder was washed with n-hexane and hot toluene, after which it was recrystallized in isopropanol to obtain 10 g (yield after drying: 42%) of $Eu(PMS)_3(Phen)_m(H_2O)_n$. The obtained white crystals were subjected to TG-DTA measurement, which confirmed the number of moles of bound water per mole of complex is 3.06.

The said white crystals were dried in vacuo for 3 hours at 140°C and a reduced pressure of 0.66 MPa to give a white powder. The resulting white powder was subjected to DSC and TG-DTA measurement, which confirmed that water had been completely removed. The results of measuring IR, NMR, and UV absorption characteristics for the obtained $Eu(PMS)_3(Phen)_8$ are given below.

IR: 3056 (C-H st.), 1512, 1423 (C=C st.), 1353 (S=O st.), 1195, 1141 (C-F st.), 1055 (S-O st.) $cm^{-1}$

$^1$R-NMR: 8.34, 7.75 (br, 8H)

UV absorption characteristics: 200 to 400 nm (Phen), 394 nm ($^7F_0 \rightarrow {}^5L_6$), 465 nm ($^7F_0$ -> $^5D_2$)

Taking into account the bidentate coordination of the Phen, the area of the signal corresponding to the hydrogen atoms of the Phen in $^1$H-NMR, and other such factors, "the coordination number of the Phen": m was assumed to be 4. The yield, the concentration of the solution used in the measurement of the luminescence characteristics described below, and so forth were found by assuming m to be 4.

Example 9

Synthesis of $Nd(PMS)_3(Phen)_m$

$Nd(PMS)_3(Phen)_m$ was obtained in the same manner as in Example 8, except that 19 g of $Nd(PMS)_3$ was used instead of $Eu(PMS)_3$ (yield after drying: 12 g (50%)). The result of IR measurement for the obtained $Nd(PMS)_3(Phen)_m$ is given below.

IR: 3054 (C-H st.), 1511, 1423 (C=C st.), 1351 (S=O st.), 1193, 1140 (C-F st.), 1054 (S-O st.) $cm^{-1}$

Taking into account the bidentate coordination of the Phen, the area of the signal corresponding to the hydrogen atoms of the Phen in $^1$H-NMR, and other such factors, "the coordination number of the Phen": m was assumed to be 4. The yield, the concentration of the solution used in the measurement of the luminescence characteristics described below, and so forth were found by assuming m to be 4.

Example 10

Synthesis of $Eu(PES)_3(TPPO)_8$

A 10 g quantity of $C_2F_5SO_2NHSO_2C_2F_5$ was dissolved in 10 mL of water, after which 2.2 g of $Eu_2O_3$ was added, and the system was refluxed for 30 minutes. A 100 mL quantity of an isopropanol solution in which 28 g of TPPO had been dissolved was added dropwise, and the system was refluxed for another 3 hours. Then, the reaction solution was cooled to room temperature, and the resulting white precipitate was collected by filtration. The obtained white precipitate was dried for 1 hour at 140°C and a pressure of 0.66 MPa to give 33.9 g of $Eu(PES)_3(TPPO)_8$ in the form of a white powder (yield: 81%). The obtained crystals were subjected to TG-TDA measurement, which revealed a weight reduction at 100°C. It is found that the number of moles of bound water per mole of complex was 0.19. The result of IR measurement of the obtained $Eu(PES)_3(TPPO)_8$ are given below.

IR: 3060 (C-H st.), 1438 (C=C st.), 1356 (S=O st.), 1192, 1122 (C-F st.), 1059 (S-O st.) $cm^{-1}$

Example 11

Synthesis of $Eu(C_6F_5SO_2NSO_2C_6F_5)_3(TPPO)_8$

$C_6F_5SO_2NHSO_2C_6F_5$ (white solid) was obtained, in the same manner as in Producing Example 2, except that $C_6F_5SO_2Cl$ was used instead of $C_4F_9SO_2F$ (yield: 24%). The results of $^{19}F$-NMR, IR, and UV absorption characteristic measurement of the resulting $C_6F_5SO_2NHSO_2C_6F_5$ are given below.

$^{19}F$-NMR: -137.07, -152.93, -161.74 ppm

IR: 1479 ($C_6F_5$), 1306 (S=O), 1247 (C-F) , 1227 (C-F), 1115 (S-O) $cm^{-1}$

UV absorption characteristics: 233, 266 nm

$Eu(C_6F_5SO_2NSO_2C_6F_5)_3$ was obtained in the same manner as in Producing Example 1, except that $C_6F_5SO_2NHSO_2C_6F_5$ obtained by the above method was used instead of $CF_3SO_2NHSO_2CF_3$. The results of $^{19}F$-NMR, IR, and UV absorption characteristics measurement of the obtained $Eu(C_6F_5SO_2NSO_2C_6F_5)_3$ are given below.

$^{19}F$-NNR: -136.00, -151.90, -161.23 ppm

IR: 1489 ($C_6F_5$), 1306 (S=O), 1248 (C-F), 1228 (C-F), 1116 (S-O) $cm^{-1}$

UV absorption characteristics: 233, 266, 394, 465 nm

$Eu(C_6F_5SO_2NSO_2C_6F_5)_3(TPPO)_8$ was also prepared in the same manner as in Example 1, except that Eu $(C_6F_5SO_2NSO_2C_6F_5)_3$ was used instead of $Eu(PMS)_3$ in addition to using $C_6F_5SO_2NHSO_2C_6F_5$

Comparative Example A

Synthesis of $Eu(PMS)_3(DMSO\text{-}d_6)_8$

A 6.2 g quantity of $Eu(PMS)_3$ was dissolved in 7.7 g of DMSO-$d_6$, and the system was thoroughly concentrated by distilling off the DMSO-$d_6$ at 140°C and a reduced pressure of 0.66 MPa. Then, the crystals obtained during cooling were filtered off and dried in vacuo to obtain 10 g of $Eu(PMS)_3(DMSO\text{-}d_6)_8$ in the form of white crystals (yield: 99%). The results of IR, UV absorption characteristics, and elemental analysis for the resulting $Eu(PMS)_3(DMSO\text{-}d_6)_8$ are given below.

IR: 2266, 2135 (C-D st.), 1353 (S=O st.), 1212, 1145 (C-F st.), 1059 (S-O st.) $cm^{-1}$

UV absorption characteristics: 394 nm ($^7F_0 \rightarrow {}^5L_6$), 465 nm ($^7F_0 \rightarrow {}^5D_2$)

Elemental analysis: C22D48O20N3S14F18Eu, M.W. 1665, calculated values C: 15.86, D: 5.77, N: 2.52%, found values C: 15.78, D: 2.91, N: 2.46%.

$Nd(PMS)_3(DMSO\text{-}d_6)_8$ was also prepared in the same manner as described above, except that $Nd(PMS)_3$ was used instead of $Eu(PMS)_3$.

IR: 2264, 2135 (C-D st.), 1354 (S=O st.), 1211, 1146 (C-F st.), 1059 (S-O st.) $cm^{-1}$

Comparative Producing Example 1

Eu $(HFA)_3(TPPO)_2$

$Eu(HFA)_3(TPPO)_2$, which is a β-diketone-type rare earth ternary complex, was prepared in the same manner as in Example 1, except that $Eu(HFA)_3$, which is a known β-diketone-type of rare earth complex, was used instead of $Eu(PMS)_3$.

Comparative Producing Example 2

$Nd(HFA)_3(TPPO)_2$

$Nd(HFA)_3(TPPO)_2$, which is a β-diketone-type rare earth ternary complex, was prepared in the same manner as in Example 1, except that $Nd(HFA)_3$, which is a known β-diketone-type of rare earth complex, was used instead of $Eu(PMS)_3$.

The rare earth ternary complexes obtained in Examples 1 to 10 were evaluated for their miscibility with various media (water, acetone, chloroform, toluene, epoxy resin, Nafion (trademark, made by DuPont), and polymethyl methacrylate (hereinafter referred to as PMMA). Table 1 shows the number of moles of bound water per mole of each rare earth ternary complex, and the results of evaluating miscibility. The term "bound water" in Table 1 means the number of moles of bound water per mole of complex.

Table 1. Miscibility with solvents

| | Rare earth ternary complex | Bound water | Media | | | | | | |
|---|---|---|---|---|---|---|---|---|---|
| | | | Water | Acetone | Chloroform | Toluene | Epoxy resin | Nafion | PMMA |
| Ex. 1 | $Eu(PMS)_3(TPPO)_8$ | 0.18 | × | ◎ | ◎ | ○ | ○ | ○ | ○ |
| Ex. 2 | $Nd(PMS)_3(TPPO)_8$ | 0.24 | × | ◎ | ◎ | ○ | ○ | ○ | ○ |
| Ex. 3 | $Yb(PMS)_3(TPPO)_8$ | 0.27 | × | ◎ | ◎ | ○ | ○ | ○ | ○ |
| Ex. 4 | $Tb(PMS)_3(TPPO)_8$ | 0.08 | × | ◎ | ◎ | ○ | ○ | ○ | ○ |
| Ex. 5 | $Eu(PMS)_3(DPSO)_8$ | 0.00 | × | ◎ | ○ | ○ | ○ | ○ | ○ |
| Ex. 6 | $Tb(PMS)_3(DPSO)_8$ | 0.00 | × | ◎ | ○ | ○ | ○ | ○ | ○ |
| Ex. 7 | $Nd(PMS)_3(DPSO)_8$ | 0.00 | × | ◎ | ○ | ○ | ○ | ○ | ○ |
| Ex. 8 | $Eu(PMS)_3(Phen)_m$ | 0.00 | × | ◎ | Δ | Δ | ○ | ○ | ○ |
| Ex. 9 | $Nd(PMS)_3(Phen)_m$ | 0.00 | × | ◎ | Δ | Δ | ○ | ○ | ○ |
| Ex. 10 | $Eu(PES)_3(TPPO)_8$ | 0.19 | × | ◎ | ◎ | ○ | ○ | ○ | ○ |
| CE 1 | $Eu(PMS)_3$ | 2.08 | ◎ | ◎ | × | × | Δ | Δ | × |
| CE 2 | $Nd(PMS)_3$ | 2.32 | ◎ | ◎ | × | × | Δ | Δ | × |
| CE 3 | $Eu(HFA)_3$ | 2.00 | × | ◎ | × | × | ○ | Δ | × |
| CE 4 | $Eu(HFA)_3(TPPO)_2$ | 0.00 | × | ◎ | ○ | ○ | ○ | Δ | Δ |
| CE 5 | $Nd(HFA)_3$ | 2.00 | × | ◎ | × | × | ○ | Δ | × |
| CE 6 | $Nd(HFA)_3(TPPO)_2$ | 0.00 | × | ◎ | ○ | ○ | ○ | Δ | Δ |

Note: PMMA: polymethyl methacrylate. [CE: Comparative Example]

Comparative Example 1

The miscibility of the $Eu(PMS)_3$ obtained in Producing Example 1 with various media was evaluated. Table 1 shows the results of evaluating the miscibility and the number of moles of bound water per mole of complex for Eu $(PMS)_3$.

Comparative Example 2

The miscibility of the $Nd(PMS)_3$ obtained in Producing Example 1 with various media was evaluated. Table 1 shows the results of evaluating the miscibility and the number of moles of bound water per mole of complex for Nd $(PMS)_3$.

Comparative Example 3

The miscibility of the $Eu(HFA)_3$ obtained in Comparative Producing Example 1 with various media was evaluated. Table 1 shows the results of evaluating the miscibility and the number of moles of bound water per mole of complex for $Eu(HFA)_3$.

Comparative Example 4

The miscibility of the $Eu(HFA)_3(TPPO)_2$ obtained in Comparative Producing Example 1 with various media was evaluated. Table 1 shows the results of evaluating the miscibility and the number of moles of bound water per mole of complex for $Eu(HFA)_3(TPPO)_2$.

Comparative Example 5

The miscibility of the $Nd(HFA)_3$ used in Comparative Producing Example 2 with various media was evaluated. Table 1 shows the results of evaluating the miscibility and the number of moles of bound water per mole of complex for $Nd(HFA)_3$.

Comparative Example 6

The miscibility of the $Nd(HFA)_3(TPPO)_2$ obtained in Comparative Producing Example 2 with various media was evaluated. Table 1 shows the results of evaluating the miscibility and the number of moles of bound water per mole of complex for $Nd(HFA)_3(TPPO)_2$.

Example 12

The luminescence quantum yield of an acetonitrile solution of the $Eu(PMS)_3(TPPO)_8$ obtained in Example 1 (europium ion concentration: 0.05 mol/L) was measured (excitation wavelength: 465 nm, maximum luminescence wavelength: 612 nm, luminescence wavelength used in measurement: 612 nm). The measurement results are given in Table 2.

Table 2

| | Rare earth ternary complex | Luminescence quantum yield (%) | Relative luminescence intensity |
|---|---|---|---|
| Ex. 12 | $Eu(PMS)_3(TPPO)_8$ | 5.6 | 45.1 |
| Ex. 13 | $Eu(PMS)_3(DPSO)_8$ | 32.0 | 41.7 |
| Ex. 14 | $Eu(PMS)_3(Phen)_m$ | 5.1 | 49.5 |
| Comp. Ex. 7 | $Eu(PMS)_3$ | 5.0 | 1.0 |
| Comp. Ex. 8 | $Eu(HFA)_3$ | 8.0 | 1.0 |
| Comp. Ex. 9 | $Eu(HFA)_3(TPPO)_2$ | -* | 4.6 |

*: not measured

Example 13

The luminescence quantum yield of an acetonitrile solution of the $Eu(PMS)_3(DPSO)_8$ obtained in Example 5 (europium ion concentration: 0.05 mol/L) was measured (excitation wavelength: 465 nm, maximum luminescence wavelength: 612 nm, luminescence wavelength used in measurement: 612 nm). The measurement results are given in Table 2.

Example 14

The luminescence quantum yield of an acetonitrile solution of the $Eu(PMS)_3(Phen)_m$ obtained in Example 8 (europium ion concentration assuming m = 4: 0.05 mol/L) was measured (excitation wavelength: 465 nm, maximum luminescence wavelength: 612 nm, luminescence wavelength used in measurement: 612 nm). The measurement results are given in Table 2.

Comparative Example 7

The luminescence quantum yield of an acetonitrile solution of the $Eu(PMS)_3$ obtained in Producing Example 1 (europium ion concentration: 0.05 mol/L) was measured (excitation wavelength: 465 nm, maximum luminescence wavelength: 612 nm, luminescence wavelength used in measurement: 612 nm). The measurement results are given in Table 2.

Comparative Example 8

The luminescence quantum yield of an acetonitrile solution of the $Eu(HFA)_3$ used in Producing Example 1 (europium ion concentration: 0.05 mol/L) was measured (excitation wavelength: 465 nm, maximum luminescence wavelength: 612 nm, luminescence wavelength used in measurement: 612 nm). The measurement results are given in Table 2.

Comparative Example 9

The luminescence quantum yield of an acetonitrile solution of the $Eu(HFA)_3(TPPO)_2$ obtained in Comparative Producing Example 1 (europium ion concentration: 0.05 mol/L) was measured (excitation wavelength: 465 nm, maximum luminescence wavelength: 612 nm, luminescence wavelength used in measurement: 612 nm). The measurement results are given in Table 2.

Comparative Example B

The luminescence quantum yield of an acetonitrile solution of the $Eu(PMS)_3(DMSO\text{-}d_6)_8$ obtained in Comparative Example A (europium ion concentration: 0.05 mol/L) was measured (excitation wavelength: 465 nm, maximum luminescence wavelength: 612 nm, luminescence wavelength used in measurement: 612 nm). The luminescence quantum yield was 44.0%.

The solutions prepared in Examples 12 to 14 and Comparative Examples 7 to 9 and Comparative Example B were measured for luminescence intensity at the respective maximum luminescence wavelengths.

The relative luminescence intensity of the solutions used in Examples 12 to 14 and Comparative Example B: (luminescence intensity in each of Examples 12 to 14)/(luminescence intensity in Comparative Example 7) was calculated, letting 1 be the luminescence intensity in Comparative Example 7 (solution of a rare earth complex in which the ligand Z was not coordinated).

Similarly, the relative luminescence intensity in Comparative Example 9: (luminescence intensity in Comparative Example 9)/(luminescence intensity in Comparative Example 8) was calculated, letting 1 be the luminescence intensity in Comparative Example 8 (solution of a known β-diketone-type of rare earth complex in which the ligand Z was not coordinated). These results are given in Table 2. The relative luminescence intensity of the solution used in Comparative Example B was 1.6 times.

Example 15

The luminescence quantum yield of an acetone-$d_6$ solution of the $Nd(PMS)_3(TPPO)_8$ obtained in Example 2 (neodymium ion concentration: 0.05 mol/L) was measured (excitation wavelength: 585 nm, maximum luminescence wavelength: 1064 nm, luminescence wavelength used in measurement: 1064 nm). The measurement results are given in Table 3.

Table 3

| | Rare earth ternary complex | Luminescence quantum yield (%) | Relative luminescence intensity |
|---|---|---|---|
| Ex. 15 | $Nd(PMS)_3(TPPO)_8$ | 1.1 | 1.4 |
| Ex. 16 | $Nd(PMS)_3(DPSO)_8$ | 1.3 | 1.6 |
| Comp. Ex. 10 | $Nd(PMS)_3$ | 0.8 | 1.0 |

Example 16

The luminescence quantum yield of an acetone-$d_6$ solution of the $Nd(PMS)_3(DPSO)_8$ obtained in Example 7 (neodymium ion concentration: 0.05 mol/L) was measured (excitation wavelength: 585 nm, maximum luminescence wavelength: 1064 nm, luminescence wavelength used in measurement: 1064 nm). The measurement results are given in Table 3.

Comparative Example 10

The luminescence quantum yield of an acetone-$d_6$ solution of the $Nd(PMS)_3$ obtained in Producing Example

1 (neodymium ion concentration: 0.05 mol/L) was measured (excitation wavelength: 585 nm, maximum luminescence wavelength: 1064 nm, luminescence wavelength used in measurement: 1064 nm). The measurement results are given in Table 3.

The solutions prepared in Examples 15 and 16 and Comparative Examples 10 were measured for luminescence intensity at the respective maximum luminescence wavelengths. The relative luminescence intensity in Examples 15 and 16: (luminescence intensity in each of Examples 15 and 16)/(luminescence intensity in Comparative Example 10) was calculated, letting 1 be the luminescence intensity in Comparative Example 10 (rare earth complex in which the ligand Z was not coordinated). These results are given in Table 3.

Example 17

A 1 mL quantity of refined anhydrous methyl methacrylate (MMA), 0.5 mg of AIBN, and 148 mg (0.7 wt%) as the rare earth ion concentration) of the $Eu(PMS)_3(TPPO)_8$ obtained in Example 1 were mixed, the mixture was transferred to a Pyrex tube, and the inside of the tube was deaerated and then sealed. By reacting for 5 hours at 60°C, the polymerization of MMA was carried out. The resulting PMMA containing $Eu(PMS)_3(TPPO)_8$ was taken out of the Pyrex tube, and a rod-shaped polymer matrix composition was obtained. Table 4 shows the luminescence quantum yield of this polymer matrix composition (excitation wavelength: 465 nm, maximum luminescence wavelength: 612 nm, luminescence wavelength used in measurement: 612 nm).

Table 4

| | Rare earth ternary complex | Luminescence quantum yield (%) | Relative luminescence intensity |
|---|---|---|---|
| Ex. 17 | $Eu(PMS)_3(TPPO)_8$ | 19.0 | 15466 |
| Ex. 18 | $Eu(PMS)_3(DPSO)_8$ | 39.0 | 710 |
| Ex. 19 | $Eu(PMS)_3(Phen)_m$ | - | 5566 |
| Comp. Ex 11 | $Eu(PMS)_3$ | 2.5 | 1.0 |

Example 18

A rod-shaped polymer matrix composition containing $Eu(PNS)_3(DPSO)_8$ was obtained in the same manner as in Example 17, except that 74 mg (rare earth ion concentration: 0.7 wt%) of the $Eu(PMS)_3(DPSO)_8$ obtained in Example 5 was used instead of $Eu(PMS)_3(TPPO)_8$. Table 4 shows the luminescence quantum yield of the obtained polymer matrix composition (excitation wavelength: 465 nm, maximum luminescence wavelength: 612 nm, luminescence wavelength used in measurement: 612 nm).

Example 19

A rod-shaped polymer matrix composition containing $Eu(PMS)_3(Phen)_m$ was obtained in the same manner as in Example 17, except that 70 mg (rare earth ion concentration assuming m = 4: 0.7 wt%) of the $Eu(PMS)_3(Phen)_m$ obtained in Example 8 was used instead of $Eu(PMS)_3(TPPO)_8$. Table 4 shows the luminescence quantum yield of the obtained polymer matrix composition (excitation wavelength: 465 nm, maximum luminescence wavelength: 612 nm, luminescence wavelength used in measurement: 612 nm).

Comparative Example 11

A rod-shaped polymer matrix composition containing $Eu(PMS)_3$ was obtained in the same manner as in Example 17, except that 47 mg (rare earth ion concentration: 0.7 wt%) of the $Eu(PMS)_3$ obtained in Producing Example 1 was used instead of $Eu(PMS)_3(TPPO)_8$. Table 4 shows the luminescence quantum yield of the obtained polymer matrix composition (excitation wavelength: 465 nm, maximum luminescence wavelength: 612 nm, luminescence wavelength used in measurement: 612 nm).

The polymer matrix compositions prepared in Examples 17 to 19 and Comparative Example 11 were measured for luminescence intensity at the respective maximum luminescence wavelengths. The relative luminescence intensity in Examples 17 to 19: (luminescence intensity in each of Examples 17 to 19)/(luminescence intensity in Comparative Example 11) was calculated, letting 1 be the luminescence intensity in Comparative Example 11 (rare earth complex in which the ligand Z was not coordinated). These results are given in Table 4.

Example 20

A rod-shaped polymer matrix composition containing $Nd(PMS)_3(TPPO)_8$ was obtained in the same manner as in Example 17, except that 148 mg (rare earth ion concentration: 0.7 wt%) of the $Nd(PMS)_3(TPPO)_8$ obtained in Example 2 was used instead of $Eu(PMS)_3(TPPO)_8$. Table 5 shows the luminescence quantum yield of the obtained polymer matrix composition (excitation wavelength: 585 nm, maximum luminescence wavelength: 1064 nm, luminescence wavelength used in measurement: 1064 nm).

Table 5

| | Rare earth ternary complex | Luminescence quantum yield (%) | Relative luminescence intensity |
|---|---|---|---|
| Ex. 20 | $Nd(PMS)_3(TPPO)_8$ | 0.8 | 80 |
| Ex. 21 | $Nd(PMS)_3(DPSO)_8$ | 0.9 | 90 |
| Comp. Ex. 12 | $Nd(PMS)_3$ | < 0.1 | 1.0 |

Example 21

A rod-shaped polymer matrix composition containing $Nd(PMS)_3(DPSO)_8$ was obtained in the same manner as in Example 17, except that 74 mg (rare earth ion concentration: 0.7 wt%) of the $Nd(PMS)_3(DPSO)_8$ obtained in Example 7 was used instead of $Eu(PMS)_3(TPPO)_8$. Table 5 shows the luminescence quantum yield of the obtained polymer matrix composition (excitation wavelength: 585 nm, maximum luminescence wavelength: 1064 nm, luminescence wavelength used in measurement: 1064 nm).

Comparative Example 12

A rod-shaped polymer matrix composition containing $Nd(PMS)_3$ was obtained in the same manner as in Example 17, except that 47 mg (rare earth ion concentration: 0.7 wt%) of the $Nd(PMS)_3$ obtained in Producing Example 1 was used instead of $Eu(PMS)_3(TPPO)_8$. Table 5 shows the luminescence quantum yield of the obtained polymer matrix composition (excitation wavelength: 585 nm, maximum luminescence wavelength: 1064 nm, luminescence wavelength used in measurement: 1064 nm).

The polymer matrix compositions prepared in Examples 20 and 21 and Comparative Example 12 were measured for luminescence intensity at the respective maximum luminescence wavelengths. The relative luminescence intensity in Examples 20 and 21: (luminescence intensity in each of Examples 20 and 21) / (luminescence intensity in Comparative Example 12) was calculated, letting 1 be the luminescence intensity in Comparative Example 12 (rare earth complex in which the ligand Z was not coordinated). These results are given in Table 5.

The rare earth ternary complexes of the present invention had remarkably improved luminescence intensity as compared to the complexes prior to the introduction of the ligand Z. For example, the europium-based ternary complexes of the present invention underwent an increase in relative luminescence intensity of up to approximately 50 times in solution. This is clear from a comparison of Examples 12 to 14 with Comparative Example 7 in Table 2.

In contrast, with the known β-diketone-type rare earth ternary complexes, even with the ternary complexes in which TPPO was introduced, the luminescence intensity only increased about 4.6 times compared to before introduction

(Comparative Examples 8 and 9).

According to the present invention, the relative luminescence intensity was increased extremely effectively.

Meanwhile, the relative luminescence intensity in a polymer matrix composition was evaluated in Table 4. Compared to the complexes having no ligand Z, the complexes of the present invention had over 10,000 times the luminescence intensity, and it can be seen that the effect of a ternary complex is remarkable.

Furthermore, it is obvious from Table 1 that the rare earth ternary complexes of the present invention exhibit excellent miscibility with various media. Thus, producing a ternary complex imparts miscibility with a wide range of media. According to the present invention, a rare earth complex that is useful as an optically functional material can be dispersed in many different kinds of media including various polymers.

Example 22

A 50 mg quantity of the $Eu(PMS)_3(Phen)_m$ obtained in Example 8 and 450 mg of polycarbonate were dissolved in 3 mL of methylene chloride and then uniformly stirred to obtain a polymer solution. This polymer solution was then cast onto a glass plate, after which it was dried in vacuo overnight to remove the solvent, forming a polycarbonate film containing $Eu(PMS)_3(Phen)_m$, which is about 6 um thick, on the glass plate. This polycarbonate film was peeled away from the glass plate, after which this thin film was hit with a metal rod, and it was confirmed visually that this film, though thin, emits red light.

Comparative Example 13

A polycarbonate film containing $Eu(PMS)_3$, which is about 6 μm thick, was formed on a glass plate in the same manner as in Example 22, except that 42 mg of the $Eu(PMS)_3$ prepared in Producing Example 1 was used instead of $Eu(PMS)_3(Phen)_m$.

After the polycarbonate film was peeled from the glass plate, this thin film was hit with a metal rod, but no light emission was noted.

INDUSTRIAL APPLICABILITY

The rare earth ternary complex of the present invention has high luminescence intensity and conversion efficiency. The rare earth ternary complex of the present invention is useful in applications such as lenses, pressure sensors, or the like, where it is used as an optically functional material a luminescent material, laser material or the like. It can therefore be used in optical devices such as CD players, optical disks, facsimiles, remote controls, copy machines, laser printers, large displays, medical lasers, laser machining and instrumentation, printing devices, and the like. More specifically, the rare earth ternary complex of the present invention can be applied to laser elements, light emitting diodes, liquid crystals, optical fibers, photodetectors, solar cells, and so forth.

The ternary complex of the present invention can be dispersed or suspended in various kinds of plastic products, making it possible to look through the contents or the internal structure, and can be used as a molding material capable of emitting light. It is also useful as a material for traffic signals, automotive light reflectors, and other such a material for transportation-related indicators, as a material for decorative products capable of emitting light, and so on.

## Claims

**1.** A rare earth ternary complex represented by the following formula (1):

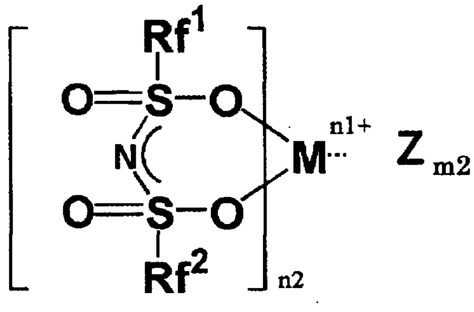

(1)

wherein M is a rare earth atom; n1 is 2 or 3; n2 is 2, 3, or 4; $Rf^1$ and $Rf^2$ are the same or different and are each an aliphatic group having 1 to 22 carbon atoms and containing no hydrogen atoms, an aromatic group containing no hydrogen atoms, or an aromatic heterocyclic group containing no hydrogen atoms; Z is at least one ligand selected from the group consisting of (A) to (D) below; m2 is an integer from 1 to 10 when Z is at least one ligand selected from the group consisting of (A) to (C) below, and is an integer from 1 to 5 when Z is at least one ligand selected from among (D) :

(A) a ligand represented by the following formula (A):

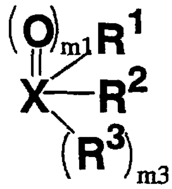

(A)

wherein $R^1$, $R^2$, and $R^3$ are the same or different and are each a hydrogen atom, a deuterium atom, an alkyl group having 1 to 20 carbon atoms, an alkyloxy group having 1 to 20 carbon atoms, an aromatic group, or an aryloxy group, and these groups may be substituted with deuterium, provided that at least one of $R^1$, $R^2$, and $R^3$ is an aromatic group or an aryloxy group; X is a phosphorus or sulfur atom; m1 is 0 or 1; and when X is a phosphorus atom, m3 is 1, and when X is a sulfur atom, m3 is 0,

(B) a ligand represented by the following formula (B):

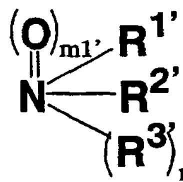

(B)

wherein $R^{1'}$, $R^{2'}$, and $R^{3'}$ are the same or different and are each a hydrogen atom, a deuterium atom, an alkyl group having 1 to 20 carbon atoms, an alkyloxy group having 1 to 20 carbon atoms, or an aromatic group, and these groups may be substituted with deuterium, provided that at least one of $R^{1'}$, $R^{2'}$, and $R^{3'}$ is an aromatic group; $m^{1'}$ is 0 or 1; and m3' is 1,

(C) a ligand which is a nitrogen-containing aromatic compound with monodentate coordination to M, and

(D) a ligand which is a nitrogen-containing aromatic compound with bidentate coordination to M.

**2.** The rare earth ternary complex according to Claim 1, wherein Z is a ligand represented by formula (A).

**3.** The rare earth ternary complex according to Claim 2, wherein formula (A) is as follows:

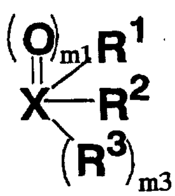

(A)

wherein $R^1$, $R^2$, and $R^3$ are the same or different and are each a hydrogen atom, an alkyl group having 1 to 20 carbon atoms, an alkyloxy group having 1 to 20 carbon atoms, an aromatic group, or an aryloxy group, and these groups may be substituted with deuterium, provided that at least one of $R^1$, $R^2$, and $R^3$ is an aromatic group or an aryloxy group; X is a phosphorus or sulfur atom; m1 is 0 or 1; and when X is a phosphorus atom, m3 is 1, and when X is a sulfur atom, m3 is 0.

**4.** The rare earth ternary complex according to Claim 1, which is obtainable by a preparing method comprising the step of mixing a rare earth complex represented by formula (2) :

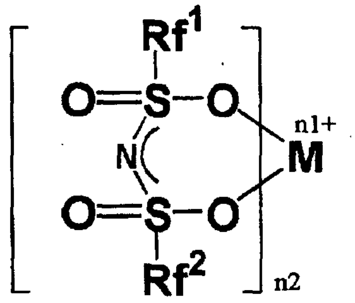 (2)

wherein M, n1, n2, $Rf^1$ and $Rf^2$ are as defined in Claim 1; and at least one compound selected from the group consisting of (A) to (D) below:

(A) a compound represented by the following formula (3) :

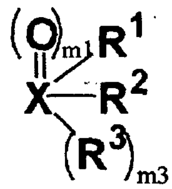 (3)

wherein $R^1$, $R^2$, and R3 are the same or different and are each a hydrogen atom, a deuterium atom, an alkyl group having 1 to 20 carbon atoms, an alkyloxy group having 1 to 20 carbon atoms, an aromatic group, or an aryloxy group, and these groups may be substituted with deuterium, provided that at least one of $R^1$, $R^2$, and $R^3$ is an aromatic group or an aryloxy group; X is a phosphorus or sulfur atom; m1 is 0 or 1; and when X is a phosphorus atom, m3 is 1, and when X is a sulfur atom, m3 is 0,

(B) a compound represented by the following formula (B'):

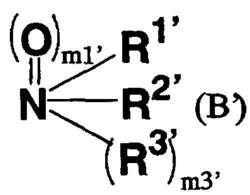 (B')

wherein $R^{1'}$, $R^{2'}$, and $R^{3'}$ are the same or different and are each a hydrogen atom, a deuterium atom, an alkyl group having 1 to 20 carbon atoms, an alkyloxy group having 1 to 20 carbon atoms, or an aromatic group, and these groups may be substituted with deuterium, provided that at least one of $R^{1'}$, $R^{2'}$, and $R^{3'}$ is an aromatic group; m1' is 0 or 1; and m3' is 1,

(C) a nitrogen-containing aromatic compound with monodentate coordination to M, and

(D) a nitrogen-containing aromatic compound with bidentate coordination to M

**5.** The rare earth ternary complex according to Claim 2, wherein at least one of $R^1$, $R^2$, and $R^3$ in formula (A) is an aromatic group.

**6.** The rare earth ternary complex according to Claim 2, wherein $R^1$, $R^2$, and $R^3$ in formula (A) are phenyl groups.

**7.** The rare earth ternary complex according to Claim 1, wherein said complex is represented by formula (4):

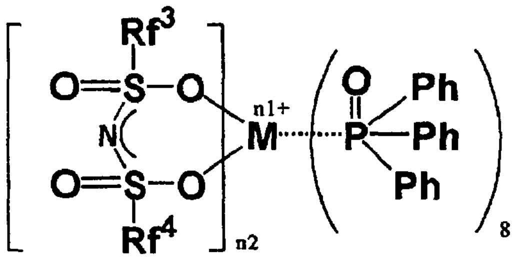

(4)

wherein M, n1, and n2 are as defined in Claim 1; $Rf^3$ and $Rf^4$ are the same or different and are each a perfluoroalkyl group having 1 to 4 carbon atoms; and Ph is a phenyl group.

**8.** The rare earth ternary complex according to Claim 1, wherein said complex is represented by formula (5):

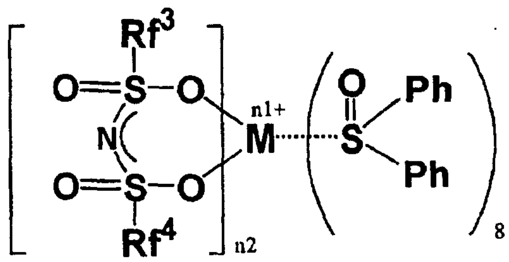

(5)

wherein M, n1, and n2 are as defined in Claim 1; $Rf^3$ and $Rf^4$ are the same or different and are each a perfluoroalkyl group having 1 to 4 carbon atoms; and Ph is a phenyl group.

**9.** The rare earth ternary complex according to Claim 1, 7, or 8, wherein M is a rare earth atom selected from the group consisting of Nd, Eu, Tb, and Yb.

**10.** A composition comprising:

a rare earth binary complex represented by formula (2):

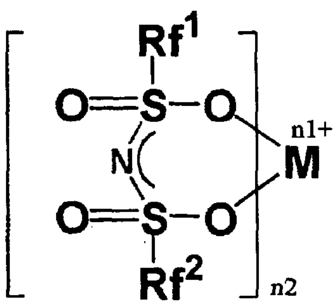

(2)

wherein M, n1, n2, $Rf^1$ and $Rf^2$ are as defined in Claim 1; and at least one compound selected from the group consisting of (A) to (D) below:

(A) a compound represented by the following formula (3) :

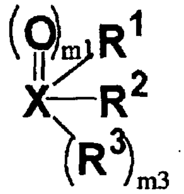

(3)

wherein $R^1$, $R^2$, and $R^3$ are the same or different and are each a hydrogen atom, a deuterium atom, an alkyl group having 1 to 20 carbon atoms, an alkyloxy group having 1 to 20 carbon atoms, an aromatic group, or an aryloxy group, and these groups may be substituted with deuterium, provided that at least one of $R^1$, $R^2$, and $R^3$ is an aromatic group or an aryloxy group; X is a phosphorus or sulfur atom; m1 is 0 or 1; and when X is a phosphorus atom, m3 is 1, and when X is a sulfur atom, m3 is 0,

(B) a compound represented by the following formula (B'):

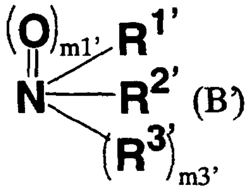

(B')

wherein $R^{1'}$, $R^{2'}$, and $R^{3'}$ are the same or different and are each a hydrogen atom, a deuterium atom, an alkyl group having 1 to 20 carbon atoms, an alkyloxy group having 1 to 20 carbon atoms, or an aromatic group, and these groups may be substituted with deuterium, provided that at least one of $R^{1'}$, $R^{2'}$, and $R^{3'}$ is an aromatic group; m1' is 0 or 1; and m3' is 1,

(C) a nitrogen-containing aromatic compound with monodentate coordination to M, and

(D) a nitrogen-containing aromatic compound with bidentate coordination to M.

**11.** The composition according to Claim 10, further comprising a solvent.

**12.** The composition according to Claim 10, further comprising a polymer matrix or a monomer which serves as the raw material for a polymer matrix.

**13.** An optically functional material comprising the rare earth ternary complex according to Claim 1.

**14.** An optically functional material comprising the composition according to Claim 10.

## INTERNATIONAL SEARCH REPORT

International application No.
PCT/JP01/07931

A. CLASSIFICATION OF SUBJECT MATTER
Int.Cl[7] C07C311/48, 317/14, C07D291/02, C07F5/00, 9/53, C09K11/06, G02B1/04, 6/00

According to International Patent Classification (IPC) or to both national classification and IPC

B. FIELDS SEARCHED

Minimum documentation searched (classification system followed by classification symbols)
Int.Cl[7] C07C311/00, 317/00, C07D291/00, C07F5/00, 9/00, C09K11/00, G02B1/00, 6/00

Documentation searched other than minimum documentation to the extent that such documents are included in the fields searched

Electronic data base consulted during the international search (name of data base and, where practicable, search terms used)
REGISTRY (STN), CA (STN)

C. DOCUMENTS CONSIDERED TO BE RELEVANT

| Category* | Citation of document, with indication, where appropriate, of the relevant passages | Relevant to claim No. |
|---|---|---|
| A | JP 2000-63682 A (New Japan Chemical Co., Ltd.), 29 February, 2000 (29.02.00), pages 3 to 9 (Family: none) | 1-14 |
| A | EP 970959 A1 (New Japan Chemical Co.), 12 January, 2000 (12.01.00), & WO 98/40388 A1 | 1-14 |
| A | JP 1-256584 A (Idemitsu Kosan Co., Ltd.), 13 October, 1989 (13.10.89), pages 2 to 4 (Family: none) | 1-14 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 December, 2001 (10.12.01) | 18 December, 2001 (18.12.01) |
| Name and mailing address of the ISA/ Japanese Patent Office | Authorized officer |
| Facsimile No. | Telephone No. |

Form PCT/ISA/210 (second sheet) (July 1992)